# EUROPEAN PATENT APPLICATION

(11) **EP 4 814 110 A1**
(43) Date of publication of application: **30.09.2026**
(21) Application number: 24890167.0
(22) Date of filing: 22.07.2024
(51) Int. Cl.: C12N 5/28, A61K 35/74, A61K 47/46, A61K 35/15, A61P 35/00, C12N 5/0786, C12N 1/20

(54) **METHOD FOR PREPARING FREEZING SHOCK-TREATED MONOCYTES OR MACROPHAGES LOADED WITH ATTENUATED SALMONELLA AND USE THEREOF**

(30) Priority: 16.11.2023 CN 202311536073
(71) Applicant: Jiangsu Targetpharma Laboratories Inc., Changzhou, Jiangsu 213164 (CN)
(72) Inventor: HUA, Zichun, Hangzhou, Jiangsu 213164 (CN); WU, Leyang, Hangzhou, Jiangsu 213164 (CN); DU, Cengzheng, Hangzhou, Jiangsu 213164 (CN)
(74) Representative: Casalonga
(86) International application number: PCT/CN2024/106720
(87) International publication number: WO 2025/102801

(57) **Abstract**

Provided are a preparation method and application of cryo-shocked monocytes or macrophages loaded with attenuated *Salmonella.* The method comprises loading engineered attenuated *Salmonella* into monocytes or macrophages, followed by cryo-shock treatment of monocytes or macrophages loaded with the strain using liquid nitrogen. The method for preparing the cryo-shocked immune cells loaded with the strain is simple and easy to operate, and solves the source problem of the monocytes or macrophages loaded with engineered attenuated *Salmonella* is solved, thus having good application prospects. This strategy improves the biosafety of bacteria-based anti-tumor therapy by avoiding bacterial exposure and heterologous stimulation; and high intratumoral strain titer promotes an anti-tumor immune response, thereby achieving stronger anti-tumor efficacy.

## Description

### TECHNICAL FIELD

The present invention relates to the field of biotechnologies, in particular to a preparation method and application of cryo-shocked monocytes or macrophages loaded with attenuated *Salmonella*.

### BACKGROUND ART

Bacteriotherapy is reshaping the landscape of tumor therapy. These therapeutic bacteria can achieve preferential colonization in solid tumor sites after injection due to their facultative anaerobic characteristics and the unique features of solid tumor microenvironment, including low-oxygen environment inside solid tumors, immunosuppressive environment, and nutrients released by a large quantity of necrotic cells (Gurbatri CR et al., 2020, Sci Transl Med, 12(530); Zhou S et al., 2018, Nat Rev Cancer, 18(12): 727-43; Suh S et al., 2019, Adv Sci, 6(3): 1801309), followed by significant intratumoral immune activation (Wu L et al., Adv Drug Deliv Rev 2022,187: 114363). However, direct injection of heterologous microorganisms invariably triggers a rapid immune response in the body, leading to discomfort and potential adverse effects. Toxicity to the host from direct administration of live bacteria has been shown to limit tolerable doses and efficacy (Wu L et al., Adv Drug Deliv Rev 2022, 187: 114363; Gurbatri CR et al., Science, 2022, 378(6622): 858-864). VNP20009 is an attenuated strain of *Salmonella typhimurium* (abbreviated as VNP hereafter) that has received extensive attention for its lower biotoxicity and good preclinical antitumor effect (Clairmont C et al., 2000, J Infect Dis, 181: 1996-2002). Although the toxicity of the strain VNP20009 is greatly reduced compared with original *Salmonella* through deletion/mutation of two genes (purI and msbB), in preclinical mouse experiments, antitumor therapy with intravenous or intraperitoneal injection of VNP20009 still caused certain toxic and side effects due to the presence and accumulation of the strain in normal organs, such as liver damage, spleen enlargement, and sudden weight loss in mice. Ideal bacteria-based therapies for cancer should minimize toxic effects caused by off-target or antigenic stimulation to ensure high biocompatibility.

In recent years, several types of leukocytes, including macrophages, neutrophils and T cells, have been used as effective antitumor drug delivery carriers due to their unique chemotactic effect towards tumor regions (Xie Z et al., 2017, Small, 13(10); Xue J et al., 2017, Nat Nanotechnol, 12(7): 692-700; Huang B et al., 2015, Sci Transl Med, 7(291): 291ra94). An ideal cell carrier should be easy to prepare and rapidly accessible, which is difficult to achieve for immune cell carriers with stem cells or primary cells as primary sources. This is because such cells usually need complex culture condition and are proliferated poorly *in vivo*. Some immortalized immune cell lines, such as macrophage (MACS) lines RAW264.7, J774.1, Ana-1, iBMDM, U937, monocyte (MC) lines THP1, iBMMC, J-111, Mono-Mac-1, and JOSK-M, have the characteristics of continuous proliferation and easy culture, which can theoretically solve the problem of difficult cell acquisition in conventional cell therapy. However, these cell lines are associated with potential pathogenicity due to their capacity of continuous proliferation. Typically, the structure of live cells disintegrates upon death, leading to loss of protein and cytokines (Green DR et al., Cold Spring Harb Perspect Biol., 2015; 7(12):a006080). In addition, external stimulation that may induce cell death, such as heat or radiation, can also inactivate protein (Sanchez Y et al., Science, 1990; 248(4959):1112-1115; Prise KM et al., Lancet Oncol., 2005; 6(7):520-528). It has been shown that snap-freezing with liquid nitrogen for live tumor cells can maintain the structural integrity of the cells and biological activity of protein on membranes while enabling the live tumor cells to lose their growth capacity (Ci T et al., Sci Adv., 2020; 6(50): eabc3013; Meng J et al., Nat Commun., 2023; 14(1):4505).

Therefore, is it possible to obtain "dead" but "functional" immortalized immune cell lines through liquid nitrogen cryo-shock treatment to eliminate their pathogenicity while maintaining accessibility? Furthermore, by loading bacteria into immune cell lines followed by liquid nitrogen cryo-shock treatment, and based on the camouflage protection of the cells and the cell-dependent targeted delivery to tumors, the toxic side effects caused by off-target bacteria and direct massive exposure to heterologous substances could be avoided. The stimulation of strains in immune cells further promotes the production of intracellular anti-tumor inflammatory factors and adhesion factors, which translates to stronger anti-cancer efficacy and intratumoral enrichment.

### SUMMARY OF THE INVENTION

The objective of the present invention is to provide a reparation method and application of cryo-shocked monocytes or macrophages loaded with attenuated *Salmonella*. Specifically, it involves loading attenuated *Salmonella* into monocytes/macrophages or other immune cells *in vitro*, followed by snap freezing with liquid nitrogen to obtain cryo-shocked monocytes/macrophages loaded with attenuated *Salmonella*, and its combination with other tumor therapies.

In order to solve the above technical problems, the technical solution adopted by the present invention is as follows: in a first aspect, the present application provides a method for preparing cryo-shocked monocytes or macrophages loaded with attenuated *Salmonella*.

In a second aspect, the present application provides cryo-shocked monocytes or macrophages loaded with attenuated *Salmonella* prepared by a method for preparing cryo-shocked monocytes or macrophages loaded with attenuated *Salmonella*.

In a third aspect, the present application provides use of a method for preparing cryo-shocked monocytes or macrophages loaded with attenuated *Salmonella* in the preparation of anti-tumor drug formulations.

A method for preparing cryo-shocked monocytes or macrophages loaded with attenuated *Salmonella* includes the following steps: co-culture an immortalized monocyte line or macrophage line with attenuated *Salmonella typhimurium* at an MOI of 1-100 to obtain engineered cells loaded with attenuated *Salmonella*, followed by snap freezing with liquid nitrogen to obtain the cryo-shocked monocytes or macrophages loaded with attenuated *Salmonella*.

The monocyte/macrophage line loses its original potential pathogenicity, while the intracellular attenuated *Salmonella* can maintain the biological activity and reach a growth platform phase within 24 hours after being released again *in vitro*. Compared with injection of cryo-shocked attenuated *Salmonella* alone and a simple mixture of cryo-shocked macrophages and cryo-shocked attenuated *Salmonella*, the strategy that utilizes the camouflage protection of cryo-shocked macrophages to bacteria and targeted drug delivery to tumor achieves significantly more potent inhibition of tumor growth in mice, along with a marked extension in their survival time.

Furthermore, (1) any of monocyte lines or macrophage lines is co-incubated with the attenuated *Salmonella:*
Macrophages or macrophages in good growth condition are plated into a petri dish at 1-100×10⁵ cells per well and cultured using antibiotic-free cell culture medium. Monoclonal strains are picked from the agar plates and activated overnight in LB liquid medium. Bacterial broth grown to log phase is centrifuged at 5000-8000 rpm for 5-10 min, the supernatant is removed and the sedimented cells are resuspended in sterile saline. After adjusting the OD600 to 0.6-1.2, the bacteria are added to petri dishes plated with the cells described above (MOI 1-100) and incubated together for 20-150 min;

(2) To observe morphological changes in cells, cells are observed with the help of a microscope after staining cell nuclei with Hoechst. Changes in the percentage of broken monocytes/macrophages at different time points are recorded. Subsequently, the supernatant is removed and the sedimented cells are washed 2-3 times with sterile PBS and then incubated for 20-60 min using cell culture medium supplemented with 50-125 µg/ml gentamicin. Gentamicin is able to kill extracellular strains. The culture supernatant is removed, and the sedimented cells are resuspended and washed 2-3 times with sterile PBS. Finally, the cells are collected to obtain live monocytes or macrophages loaded with attenuated *Salmonella*. To detect the number of attenuated *Salmonella* effectively loaded in monocytes or macrophages, the number of live monocytes or macrophages loaded with attenuated *Salmonella* is detected with the help of a cell counter at various time points, and the cells are subsequently lysed with 0.5% Triton X-100 at room temperature. The lysate is diluted and applied to LB agar plates supplemented with kanamycin and incubated overnight at 37°C. The number of viable bacteria loaded in the monocytes/macrophages is counted.

(3) The live cells of the above prepared monocytes or macrophages loaded with attenuated *Salmonella* are resuspended with 500-1000 µL of serum-free cell cryopreservation solution. Subsequently, the cell suspension is snap-frozen directly in liquid nitrogen and left to stand for 6-18 hours before being removed. The cryo-shocked monocytes or macrophages loaded with attenuated *Salmonella* are obtained after thawing in a water bath at 37-42°C.

Snap-freezing shock treatment with liquid nitrogen for 6-18 hours can eliminate the pathogenicity of cells, but does not affect the tumor enrichment capacity of cells or the biological activity of intracellular attenuated *Salmonella*.

Furthermore, in step (1) or step (2), the attenuated *Salmonella typhimurium* is attenuated *Salmonella typhimurium* VNP20009 and genetically-modified strains thereof and synthetic biologically engineered strains capable of producing therapeutic proteins, including but not limited to aforementioned strains with filed invention applications (ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2; phoP/phoQ), and the immortalized monocyte line/macrophage line includes any one of THP1, iBMMC, J-111, Mono-Mac-1, JOSK-M, RAW264.7, J774.1, Ana-1, iBMD, or U937; wherein the monocytes include any one of monocyte lines THP1, iBMMC, J-111, Mono-Mac-1, or JOSK-M, and the macrophages include any one of macrophage lines RAW264.7, J774.1, Ana-1, iBMDM, or U937.

Furthermore, in step (2), the macrophage line RAW264.7 is taken as an example, after comprehensively considering the loading capacity of strains and cell integrity, a co-culture time of 60 minutes is selected as an optimal time point for preparing live CELL/VNP cells, due to high live bacterial load, with 257±27 bacteria per 100 cells, and high cell integrity of > 90%.

Cryo-shocked monocytes or macrophages loaded with attenuated *Salmonella* prepared by a preparation method according to the present invention are provided.

Use of a method for preparing cryo-shocked monocytes or macrophages loaded with attenuated *Salmonella* according to the present invention in the preparation of anti-tumor drug formulations is provided.

Furthermore, a therapeutic dose of the cryo-shocked monocytes or macrophages loaded with attenuated *Salmonella* is 0.4-40×10⁶ cells (unit: cells/mouse), and the corresponding actual quantity of attenuated *Salmonella* is 0.1-10×10⁷ CFU (unit: CFU/mouse); and the cryo-shocked monocytes or macrophages loaded with attenuated *Salmonella* are administered at a single dose. Administration is primarily via intravenous or intraperitoneal injection.

Furthermore, the drug formulations include at least one of intravenous injections, intratumoral injections, or intraperitoneal injections. The cryo-shocked monocytes or macrophages loaded with attenuated *Salmonella* are mainly administered by intravenous injection for treatment, and can also be administered via intraperitoneal injection or intratumoral injection depending on different tumor types. Compared with injection of cryo-shocked attenuated *Salmonella* alone and a simple mixture of cryo-shocked macrophages and cryo-shocked attenuated *Salmonella*, the strategy that utilizes the camouflage protection of cryo-shocked macrophages to bacteria and targeted delivery to tumor achieves significantly more potent inhibition of tumor growth in mice, along with a marked extension in their survival time. Compared with injection of cryo-shocked attenuated *Salmonella* alone, the strategy that utilizes the camouflage protection of cryo-shocked macrophages to bacteria and targeted delivery to tumor increased the titer of the intratumoral *Salmonella* by 110.9%, decreased the off-target effect of normal organs by 16.3%, and reduced liver lesion regions by 90%.

Attenuated *Salmonella typhimurium* VNP20009 and genetically-modified strains thereof delivered and released from cells to tumor tissue are key factors for exerting the anti-tumor efficacy of these drugs.

Combination of the use according to the present invention and other conventional anti-tumor drugs or methods is provided.

Beneficial effects: The method for preparing cryo-shocked immune cells loaded with strains according to the present invention is simple and easy to operate, and has good application prospects. This strategy avoids massive exposure of bacterial heterologous stimuli in the body and improves the biological safety of bacteria-based anti-tumor therapy. The high titer of intratumoral strains also promotes anti-tumor immune response, resulting in stronger anti-tumor efficacy.

Compared with the prior art, the present invention has the following advantages:
(1) Cryo-shock treatment not only removes the potential pathogenicity of original monocyte lines or macrophage lines, but also maintains their original intratumoral enrichment capacity, has almost no impact on the activity and infectivity of intracellular strains, and promotes the accumulation of bacteria in tumors. The facultative anaerobic *Salmonella typhimurium* VNP20009 and the genetically modified strains thereof and the synthetic biologically engineered strains capable of producing therapeutic proteins (including but not limited to aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2; phoP/phoQ) are selected because they can survive in immune cells such as monocytes/macrophages for a long term, cryo-shock treatment with liquid nitrogen has no significant impact on their subsequent biological activity, and the strains themselves have good tumor colonization and tumor suppression effect.
(2) Liquid nitrogen cryo-shocked immortalized monocyte lines or macrophage lines, such as macrophage lines RAW264.7, J774.1, Ana-1, iBMDM, U937, etc., and monocyte lines THP1, iBMMC, J-111, Mono-Mac-1, JOSK-M, etc., are selected because they can proliferate rapidly in vitro before cryo-shock treatment, have simple nutritional requirements and well-understood research backgrounds, enabling large-scale, rapid acquisition. The cell line can lose its pathogenicity after simple cryo-shock treatment with liquid nitrogen, achieving high biological safety.
(3) Through the method for preparing cryo-shocked immortalized monocyte lines or macrophage lines according to the present invention, such engineered cells can be rapidly obtained and prepared, and the cells are non-pathogenic. The attenuated *Salmonella* VNP20009 and the genetically modified strains thereof and the synthetic biologically engineered strains capable of producing therapeutic proteins (including but not limited to aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2; phoP/phoQ) are low-toxicity and easy-to-culture bacteria with low cost, so the present invention has prospects of large-scale application and popularization. Reuse of the liquid nitrogen cryo-shocked immortalized monocyte line or macrophage line loaded with attenuated *Salmonella* VNP20009 not only verifies its significantly improved biological safety compared with use of single strains in mouse models, but also demonstrates good anti-tumor effect.
(4) The cryo-shocked monocytes/macrophages loaded with strains are efficiently enriched inside tumors after administration, avoiding the off-target of bacteria into normal organs, and the encapsulation camouflage of the monocytes/macrophages also effectively avoids the premature exposure of bacteria. Ultimately, they indirectly inhibit tumors by releasing the intracellular bacteria to activate/modulate the immune system.

### BRIEF DESCRIPTION OF THE DRAWINGS

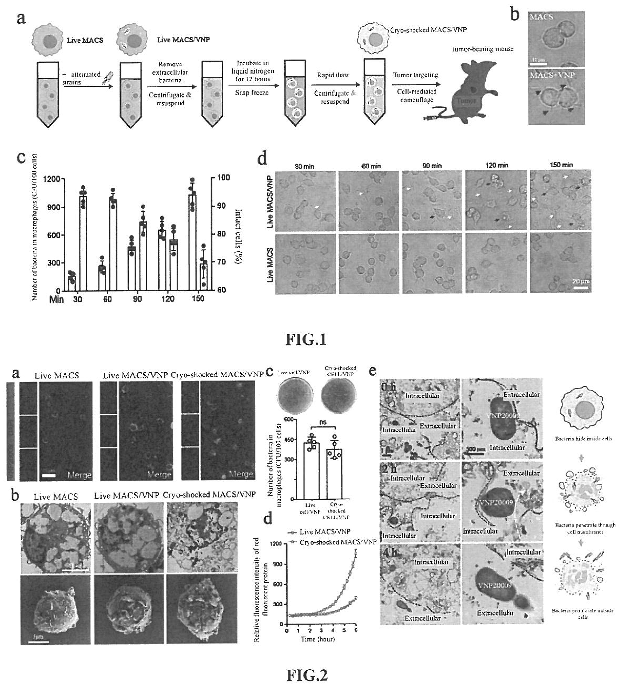

FIG. 1 shows the optimization of preparation conditions for cryo-shocked monocytes or macrophages loaded with attenuated *Salmonella* according to the present invention. FIG. 1a is a flowchart illustrating preparation of cryo-shocked monocytes or macrophages loaded with attenuated *Salmonella* according to the present invention. (A monocyte line or macrophage cell includes THP1, iBMMC, J-111, Mono-Mac-1, JOSK-M, RAW264.7, J774.1, Ana-1, iBMDM, U937. The classical macrophage line (RAW264.7) is used here as an example, but other cells are used with similar effects to RAW264.7). VNP-loaded live macrophages (live MAC*S*/VNP) were obtained by first infecting live macrophages RAW264.7 with attenuated *Salmonella* VNP20009, and then subjecting the same to freezing treatment with liquid nitrogen for 12 hours to obtain MACS/VNP cells treated with liquid nitrogen (cryo-shocked MACS/VNP). The cryo-shocked MACS/VNP cells can deliver VNP strains to tumors in a targeted manner. The resulting cryo-shocked monocytes or macrophages loaded with attenuated *Salmonella* are intravenously administered to tumor-bearing mice for targeted therapy of tumors. FIG. 1b is a diagram illustrating microscopic observation before and after co-culture of live macrophages RAW264.7 and attenuated *Salmonella* VNP20009 according to the present invention. RAW264.7 is co-cultured with VNP20009 at an MOI of 20, and nuclei are stained with nucleus dye Hoechst for convenient observation. It can be observed that VNP20009 actively infected macrophages (red arrows). FIG. 1c illustrates the total number of VNP20009 bacteria per 100 macrophages and the change of the proportion of macrophages maintaining integrity observed microscopically after co-culture of live macrophages RAW264.7 and attenuated *Salmonella* VNP20009 at an MOI of 20 for different time periods (30/60/90/120/150 minutes) according to the present invention. FIG. 1d is a diagram illustrating microscopic observation after co-culture of live macrophages RAW264.7 and attenuated *Salmonella* VNP20009 at an MOI of 20 for different time periods (30/60/90/120/150 minutes) according to the present invention. Live macrophages RAW264.7 are used as a control, nuclei are stained with nucleus dye Hoechst for ease of observation (blue), and VNP20009 around the macrophages (white arrows) and fragmented cells caused by increased loaded bacteria with prolonged co-culture time (red arrows) are observed.
FIG. 2 shows the assessment of release performance of intracellular strains of cryo-shocked monocytes or macrophages loaded with attenuated *Salmonella* according to the present invention. FIG. 2a is a confocal fluorescence microscope image of live macrophages RAW264.7 (live MACS), live macrophages loaded with attenuated *Salmonella* VNP20009 (live MACS/VNP) obtained after co-culture of live macrophages RAW264.7 and attenuated *Salmonella* VNP20009, and cryo-shocked macrophages loaded with attenuated *Salmonella* VNP20009 (cryo-shocked MACS/VNP) according to the present invention. RAW264.7 is co-cultured with VNP20009 at an MOI of 20, nuclei are stained with nucleus dye DAPI (blue), and actin is labeled with FITC-Phalloidin (green) for easier observation. It can be observed that neither strain loading nor cryo-shock significantly affected the overall integrity of macrophages, and intact VNP20009-loaded macrophages were observed (red arrows). Scale bar = 20 µm. FIG. 2b is transmission electron microscope (TEM) and scanning electron microscope (SEM) images of live macrophages RAW264.7 (live MACS), live macrophages loaded with attenuated *Salmonella* VNP20009 (live MACS/VNP), and cryo-shocked macrophages loaded with attenuated *Salmonella* VNP20009 (cryo-shocked MACS/VNP) according to the present invention. It can be observed that neither strain loading nor cryo-shock significantly affected the overall integrity of macrophages, and VNP20009-loaded macrophages were observed (red arrows). Scale bar = 5 µm; FIG. 2c is a graph illustrating the change of the number of live bacteria in live macrophages loaded with attenuated *Salmonella* VNP-RFP labeled with red fluorescent protein (RFP) (live MACS/VNP-RFP) and cryo-shocked macrophages loaded with attenuated *Salmonella* VNP-RFP labeled with RFP (cryo-shocked MACS/VNP-RFP). Representative plating results (top), ns = not significant. FIG. 2d is a graph illustrating the change of fluorescence intensity of RFP in culture plates of live macrophages loaded with attenuated *Salmonella* VNP-RFP labeled with RFP (live MACS/VNP-RFP) and cryo-shocked macrophages loaded with attenuated *Salmonella* VNP-RFP labeled with RFP (cryo-shocked MACS/VNP-RFP) monitored in real time within 6 hours according to the present invention. FIG. 2e is a TEM image (left and middle) illustrating a release process of intracellular attenuated *Salmonella* VNP20009 in cryo-shocked macrophages loaded with attenuated *Salmonella* VNP20009 (cryo-shocked MACS/VNP) and a schematic diagram (right) illustrating an intracellular bacterial release process according to the present invention, in which movement of bacteria from inside the cell to the outside is observed (white curves). Notches in surfaces of cryo-shocked MACS/VNP cells (orange arrows); extracellular proliferated bacteria (blue arrows). Scale bar: left = 1 µm, middle = 500 nm (enlarged view).

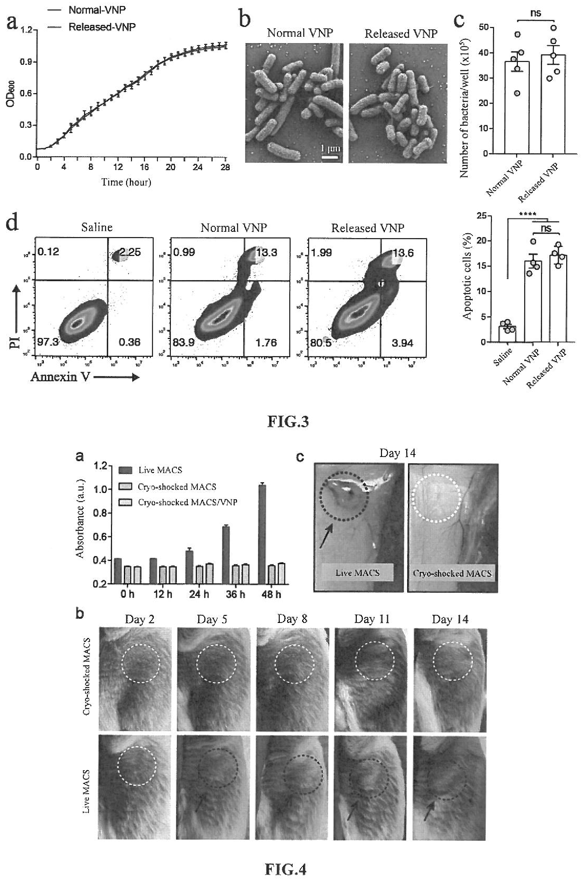

FIG. 3 shows the detection of biological activity of strains released by cryo-shocked monocytes or macrophages loaded with attenuated *Salmonella* according to the present invention. FIG. 3a is a growth curve of VNP released from cryo-shocked macrophages loaded with attenuated *Salmonella* VNP20009 (cryo-shocked MACS/VNPs) and untreated normal attenuated *Salmonella* VNP20009 in an LB liquid medium according to the present invention, where a culture temperature was 37°C. FIG. 3b is an SEM image of VNP released from cryo-shocked macrophages loaded with attenuated *Salmonella* VNP20009 (cryo-shocked MACS/VNP) and untreated normal attenuated *Salmonella* VNP20009 according to the present invention, scale bar = 1 µm. FIG. 3c is a graph showing the number of intracellular bacteria after infecting tumor cells (B16F10, LLC, 4T1, A20, H22) for 1 hour with VNP released from cryo-shocked macrophages loaded with attenuated *Salmonella* VNP20009 (cryo-shocked MACS/VNP) and with untreated normal attenuated *Salmonella* VNP20009 according to the present invention. Tumor cells were co-cultured with different VNP at an MOI of 100. After washing with PBS and treating with gentamicin for 1 hour to remove extracellular residual VNP, cells were lysed with 0.5% Triton X-100, diluted and plated to calculate the number of internalized bacteria. FIG. 3d is a representative flow cytometry plot of cells stained with Annexin V and PI after VNP is released from cryo-shocked macrophages loaded with attenuated *Salmonella* (cryo-shocked MACS/VNP) and untreated normal attenuated *Salmonella* were respectively co-incubated with H22 cells for 4 hours according to the present invention. The percentage of apoptotic cells (Annexin V+ cells) was quantitatively analyzed. H22 cells were co-cultured with different VNP at an MOI of 100. ns = not significant, ****P < 0.0001.
FIG. 4 shows the assessment of potential pathogenicity of cryo-shocked monocyte lines or macrophage lines according to the present invention. FIG. 4a illustrates cell viability of live macrophages RAW264.7 (live MACS), cryo-shocked macrophages RAW264.7 (cryo-shocked MACS), and cryo-shocked macrophages loaded with attenuated *Salmonella* VNP20009 (cryo-shocked MACS/VNP) at different time points (0/12/24/36/48 hours) according to the present invention. Cell viability was detected through a CCK8 method. a.u. is arbitrary unit. FIG. 4b illustrates analysis of biological activity of live macrophages RAW264.7 (live MACS) and cryo-shocked macrophages loaded with attenuated *Salmonella* VNP20009 (cryo-shocked MACS) at different time points (2/5/8/11/14 days) after in vivo inoculation according to the present invention. Mass caused by continuous proliferation of live macrophages is observed (black arrows). FIG. 4c is a comparison of cell proliferation activity of live macrophages RAW264.7 (live MACS) and cryo-shocked macrophages loaded with attenuated *Salmonella* VNP20009 (cryo-shocked MACS) 14 days after in vivo inoculation according to the present invention. Regions within dotted circles represent inoculation sites.

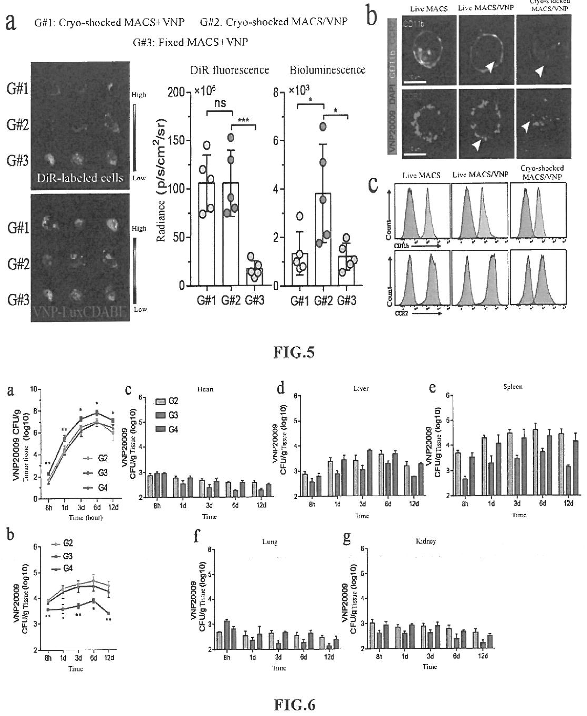

FIG. 5 shows the assessment of intratumoral enrichment performance of cryo-shocked monocytes or macrophages loaded with attenuated *Salmonella* according to the present invention. FIG. 5a is representative DIR fluorescence and bioluminescence images (left) of mouse tumors in each group and statistical graphs (right) of DIR fluorescence and bioluminescence intensity of mouse tumors in each group after administration to H22 tumor-bearing mice through different treatment methods (G#1 to G#3) for 8 hours according to the present invention. Cells were labeled with DIR to emit near infrared fluorescence, and a strain VNP-LuxCDABE used spontaneously emitted bioluminescence. G#1: a simple mixture of cryo-shocked and DIR-labeled macrophages RAW264.7 and attenuated *Salmonella* VNP-LuxCDABE; G#2: cryo-shocked and DIR-labeled macrophages loaded with attenuated *Salmonella* VNP-LuxCDABE; G#3: a simple mixture of paraformaldehyde-fixed and DIR-labeled macrophages RAW264.7 and attenuated *Salmonella* VNP-LuxCDABE. ns = not significant, *P < 0.05, ***P < 0.001. FIG. 5b is a confocal fluorescence microscope image of live macrophages RAW264.7 (live MACS), live macrophages RAW264.7 loaded with attenuated *Salmonella* VNP-RFP labeled with RFP (live MACS/VNP-RFP) obtained after co-culture of live macrophages RAW264.7 and attenuated *Salmonella* VNP-RFP labeled with RFP, and cryo-shocked macrophages loaded with attenuated *Salmonella* VNP-RFP labeled with RFP (cryo-shocked MACS/VNP-RFP) according to the present invention. The expression of CD11b (yellow) and CCR2 (green) of cells in each group, as well as the morphology of intact bacteria within macrophages (white arrows) are observed. Scale bar = 10 µm. FIG. 5c is a representative flow cytometry plots illustrating the expression of CD11b protein (top) and CCR2 protein (bottom) in live macrophages RAW264.7 (live MACS), live macrophages loaded with attenuated *Salmonella* VNP20009 (live MACS/VNP), and cryo-shocked macrophages loaded with attenuated *Salmonella* VNP20009 (cryo-shocked MACS/VNP) according to the present invention.
FIG. 6 shows the evaluation of the enhanced tumor-targeting performance of intracellular bacteria mediated by cryo-shocked monocytes or macrophages loaded with attenuated *Salmonella* for intracellular strains according to the present invention. FIG. 6a shows a relationship between different post-dose time points (8 hours/1 day/3 days/6 days/12 days) and the number of bacteria in tumors after administration to H22 tumor-bearing mice through different treatment methods (G2 to G4) according to the present invention. G2: cryo-shocked attenuated *Salmonella* (cryo-shocked VNP); G3: cryo-shocked macrophages loaded with attenuated *Salmonella* (cryo-shocked MACS/VNP); and G4: a simple mixture of cryo-shocked macrophages RAW264.7 and attenuated *Salmonella* (cryo-shocked MACS + cryo-shocked VNP). The same group descriptions apply below. *P < 0.05, **P < 0.01. FIG. 6b shows a relationship between normal organs (including hearts, livers, spleens, lungs, kidneys) and the number of VNP20009 bacteria of mice in each group at specific time points (8 hours/1 day/3 days/6 days/12 days) after administration to H22 tumor-bearing mice through different treatment methods according to the present invention. FIG. 6c-g are statistical curves illustrating titers of VNP20009 bacteria at the hearts (c), livers (d), spleens (e), lungs (f), and kidneys (g) of mice in each group at specific time points (8 hours/1 day/3 days/6 days/12 days) after administration to H22 tumor-bearing mice through different treatment methods according to the present invention.

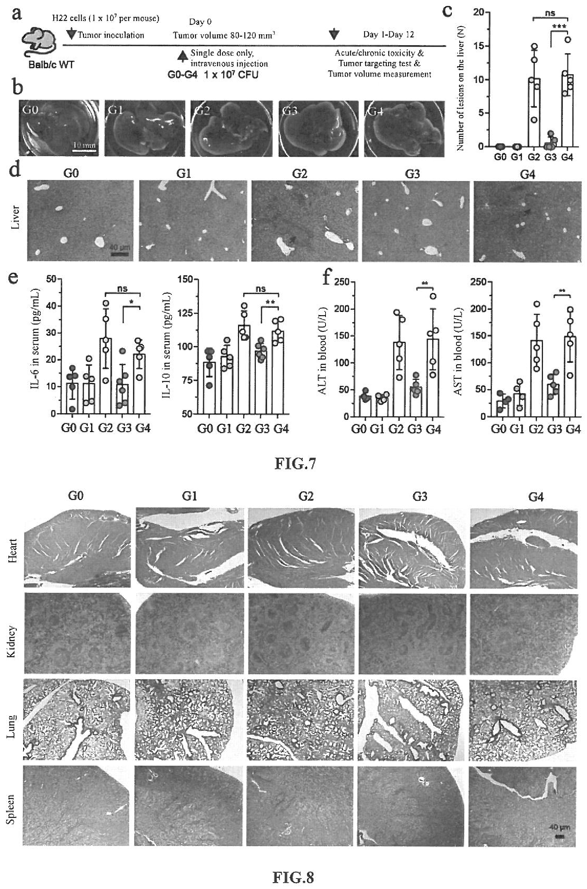

FIG. 7 shows the assessment of in vivo biological safety of cryo-shocked monocytes or macrophages loaded with attenuated *Salmonella* according to the present invention. FIG. 7a is a schematic diagram illustrating safety evaluation, tumor targeting, and therapeutic experiment of different treatment methods (G0 to G4) in H22 tumor-bearing mice according to the present invention. G0: saline group; G1: cryo-shocked macrophages RAW264.7; G2: cryo-shocked attenuated *Salmonella*; G3: cryo-shocked macrophages loaded with attenuated *Salmonella* VNP20009; and G4: a simple mixture of cryo-shocked macrophages RAW264.7 and attenuated *Salmonella*. The same group descriptions apply below. FIG. 7b is a representative image of inflammatory lesions in livers of mice in each group after administration to H22 tumor-bearing mice through different treatment methods (G0 to G4) for one day according to the present invention. Sites of pathological damage in the livers (black arrows) are observed. Scale bar =10 mm. FIG. 7c is a bar graph of the number of inflammatory lesions in the livers of mice in each group after administration to H22 tumor-bearing mice through different treatment methods (G0 to G4) for one day according to the present invention. ns = not significant, ***P < 0.001. FIG. 7d shows representative close-up H&E staining images of inflammatory lesions in the livers of mice in each group after administration to H22 tumor-bearing mice through different treatment methods (G0 to G4) for one day according to the present invention. Obvious sites of liver injury (black arrows) are observed. Scale bar = 40 µm. FIG. 7e illustrates concentrations of IL-6 (left) and IL-10 (right) in peripheral blood serum of mice in each group after administration to H22 tumor-bearing mice through different treatment methods (G0 to G4) for one day according to the present invention. ns = not significant, *P < 0.05, **P < 0.01; FIG. 7f illustrates levels of alanine aminotransferase (ALT) and aspartate aminotransferase (AST) in peripheral blood of mice in each group after administration to H22 tumor-bearing mice through different treatment methods (G0 to G4) for one day according to the present invention. ** P < 0.01.
FIG. 8 shows the assessment of influences of cryo-shocked monocytes or macrophages loaded with attenuated *Salmonella* on conventional organs according to the present invention. FIG. 8 is a close-up image illustrating representative H&E staining of heart, kidney, lung and spleen slices of mice in each group after administration to H22 tumor-bearing mice through different treatment methods (G0 to G4) according to the present invention. G0: saline group; G1: cryo-shocked macrophages RAW264.7; G2: cryo-shocked attenuated Salmonella; G3: cryo-shocked macrophages loaded with attenuated *Salmonella* VNP20009; and G4: a simple mixture of cryo-shocked macrophages RAW264.7 and attenuated *Salmonella*. Scale bar = 40 µm.

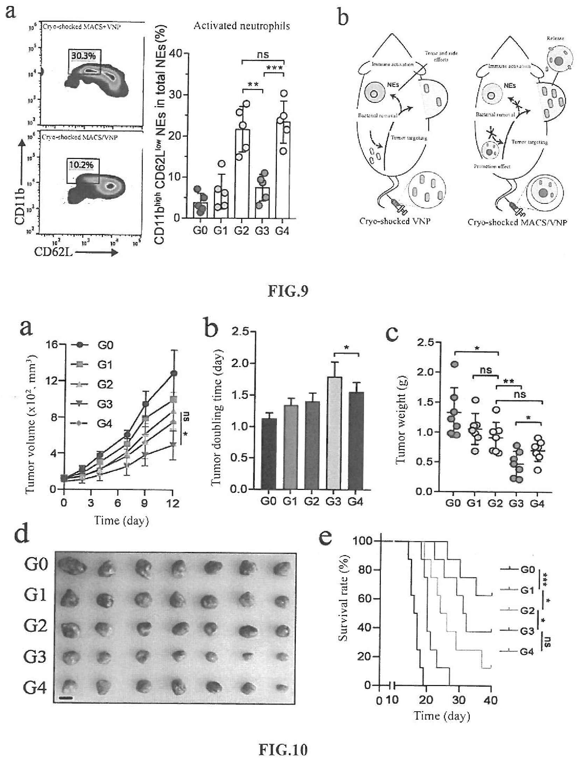

FIG. 9 shows preliminary investigation of a mechanism for improving biological safety performance in vivo of cryo-shocked monocytes or macrophages loaded with attenuated *Salmonella* according to the present invention. FIG. 9a is a representative flow comparison diagram (left) and a bar statistical curve (right) of the change of percentages of activated neutrophils (CD11b high expression, CD62L low expression) in peripheral blood after administration to H22 tumor-bearing mice for an hour through different treatment methods (G0 to G4) for one day according to the present invention. G0: saline group; G1: cryo-shocked macrophages RAW264.7; G2: cryo-shocked attenuated *Salmonella*; G3: cryo-shocked macrophages loaded with attenuated *Salmonella*; and G4: a simple mixture of cryo-shocked macrophages RAW264.7 and attenuated *Salmonella.* ns = not significant, **P < 0.01, ***P < 0.001. FIG. 9b is a schematic diagram of cryo-shocked macrophage RAW264.7-mediated tumor-targeted delivery of the VNP strain according to the present invention. The protection by cryo-shocked macrophage RAW264.7 (cryo-shocked MACS) avoids neutrophil activation triggered by exposed bacteria (red cross) and enables intratumoral targeted release of intracellular bacteria with the help of macrophages.
FIG. 10 shows the assessment of in vivo anti-tumor effects of cryo-shocked monocytes or macrophages loaded with attenuated *Salmonella* according to the present invention. FIG. 10a is a statistical curve of tumor volumes at specific time points (0/3/6/9/12 days) after administration to H22 tumor-bearing mice through different treatment methods (G0 to G4) according to the present invention. G0: saline group; G1: cryo-shocked macrophages RAW264.7; G2: cryo-shocked attenuated *Salmonella*; G3: cryo-shocked macrophages loaded with attenuated *Salmonella*; and G4: a simple mixture of cryo-shocked macrophages RAW264.7 and attenuated *Salmonella*. The same group descriptions apply below. ns = not significant, *P < 0.05. FIG. 10b is a comparison of tumor doubling time after administration to H22 tumor-bearing mice through different treatment methods (G0 to G4) according to the present invention. *P < 0.05. FIG. 10c is a statistical curve of tumor weights of mice in each group after administration to H22 tumor-bearing mice through different treatment methods (G0 to G4) for 12 days according to the present invention. ns = not significant, *P < 0.05, **P < 0.01. FIG. 10d is a photographic image of tumors after administration to H22 tumor-bearing mice through different treatment methods (G0 to G4) for 12 days according to the present invention. Scale bar = 10 mm. FIG. 10e is a survival curve of mice after administration to H22 tumor-bearing mice through different treatment methods (G0 to G4) according to the present invention. When reaching a humane endpoint, mice were euthanized. ns = not significant, *P < 0.05, ***P < 0.001.

### DETAILED DESCRIPTION OF THE EMBODIMENTS

The present invention will be described in details with reference to drawings and specific embodiments below. However, it is easily understood by those skilled in the art that the contents described by examples are merely used for describing the present invention, and shall not and will not limit the present invention described in the claims in detail. The examples without specific conditions noted will be carried out according to conventional conditions or conditions suggested by manufacturers.

In the present application, the term "and/or" describes the association relationship of associated objects, indicating that there may be three relationships, for example, A and/or B, which may denote the following: only A exists, both A and B exist, and only B exists. A and B may be singular or plural. The character "/" generally indicates that the relationship between associated objects before and after it is an "or" relationship.

The terms "first" and "second" are only intended for description and used for distinguishing purposes, such as substances, and shall not be understood as indication or implication of relative importance or implicit indication of the number of indicated technical features. For example, first XX may also be referred to as second XX, and similarly, second XX may also be referred to as first XX, without departing from the scope of the examples of the present application. Thus, the features limited with "first" and "second" can definitely or implicitly include one or more of the features.

In a first aspect of examples of the present application, a method for preparing cryo-shocked monocytes or macrophages loaded with attenuated *Salmonella* is provided, including the following steps: co-culture an immortalized monocyte line or macrophage line (including THP1, iBMMC, J-111, Mono-Mac-1, JO*S*K-M, RAW264.7, J774.1, Ana-1, iBMDM, U937, etc.) with attenuated *Salmonella typhimurium* at an MOI of 1-100 to obtain engineered cells loaded with attenuated Salmonella, followed by snap freezing with liquid nitrogen to obtain the cryo-shocked monocytes or macrophages loaded with attenuated *Salmonella*.

The monocyte/macrophage line loses its original potential pathogenicity, while the intracellular attenuated *Salmonella* can maintain biological activity and reach a growth platform phase within 24 hours after being released again *in vitro*. Compared with injection of cryo-shocked attenuated *Salmonella* alone and a simple mixture of cryo-shocked monocyte line or macrophage line and cryo-shocked attenuated *Salmonella*, the strategy that utilizes the camouflage protection of cryo-shocked monocytes or macrophages to bacteria and targeted delivery to tumor achieves significantly more potent inhibition of tumor growth in mice, along with a marked extension in their survival time.

The attenuated *Salmonella typhimurium* is attenuated *Salmonella typhimurium* VNP20009 and genetically-modified strains thereof and synthetic biologically-engineered strains capable of producing therapeutic proteins (including but not limited to aforementioned strains with filed invention applications (ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2; phoP/phoQ); and the monocyte lines/macrophage lines with immortalized colonization include, but are not limited to macrophage lines RAW264.7, J774.1, Ana-1, iBMDM U937, etc. and monocyte lines THP1, iBMMC, J-111, Mono-Mac-1, JOSK-M, etc.

Snap-freezing shock treatment with liquid nitrogen for 6-18 hours can eliminate the pathogenicity of monocytes or macrophages, but does not affect the tumor enrichment capacity of cells or the biological activity of intracellular attenuated *Salmonella*.

In some examples, a method for preparing cryo-shocked monocytes or macrophages loaded with attenuated *Salmonella* according to the present invention includes the following steps:
(1) any of monocyte lines or macrophage lines is co-incubated with the attenuated *Salmonella* strain VNP20009:
   Macrophages or macrophages in good growth condition are plated into a petri dish at 1-100×10⁵ cells per well and cultured using antibiotic-free cell culture medium. Monoclonal strains are picked from the agar plates and activated overnight in LB liquid medium. Bacterial broth grown to log phase is centrifuged at 5000-8000 rpm for 5-10 min, the supernatant is removed and the sedimented cells are resuspended in sterile saline. After adjusting the OD600 to 0.6-1.2, the bacteria are added to petri dishes plated with the cells described above (MOI 1-100) and incubated together for 20-150 min;
(2) To observe morphological changes in cells, cells are observed with the help of a microscope after staining cell nuclei with Hoechst. Changes in the percentage of broken monocytes/macrophages at different time points are recorded. Subsequently, the supernatant is removed and the sedimented cells are washed 2-3 times with sterile PBS and then incubated for 20-60 min using cell culture medium supplemented with 50-125 µg/ml gentamicin. Gentamicin is able to kill extracellular strains. The culture supernatant is removed, and the sedimented cells are resuspended and washed 2-3 times with sterile PBS. Finally, the cells are collected to obtain live monocytes/ macrophages loaded with attenuated *Salmonella*. To detect the number of attenuated *Salmonella* effectively loaded in monocytes/macrophages, the number of live monocytes or macrophages loaded with attenuated *Salmonella* is detected with the help of a cell counter at various time points, and the cells are subsequently lysed with 0.5% Triton X-100 at room temperature. The lysate is diluted and applied to LB agar plates supplemented with kanamycin and incubated overnight at 37°C. The number of viable bacteria loaded in the monocytes/macrophages is counted.
(3) The live cells of the above prepared monocytes/macrophages loaded with attenuated *Salmonella* are resuspended with 500-1000 µL of serum-free cell cryopreservation solution. Subsequently, the cell suspension is snap-frozen directly in liquid nitrogen and left to stand for 6-18 hours before being removed. The cryo-shocked monocytes or macrophages loaded with attenuated *Salmonella* are obtained after thawing in a water bath at 37-42°C.

Snap-freezing shock treatment with liquid nitrogen for 6-18 hours can eliminate the pathogenicity of cells, but does not affect the tumor enrichment capacity of cells or the biological activity of intracellular attenuated *Salmonella*.

In a second aspect of examples of the present application, cryo-shocked monocytes or macrophages loaded with attenuated *Salmonella* prepared by a method for preparing cryo-shocked monocytes or macrophages loaded with attenuated *Salmonella* are provided.

In a third aspect of examples of the present application, use of a method for preparing cryo-shocked monocytes or macrophages loaded with attenuated *Salmonella* in the preparation of anti-tumor drug formulations is provided.

A therapeutic dose of the cryo-shocked monocytes or macrophages loaded with attenuated *Salmonella* is 0.4-40×10⁶ cells, with a unit of cells/mouse, and the corresponding actual quantity of attenuated *Salmonella* is 0.1-10×10⁷ CFU, with a unit of CFU/mouse. The cryo-shocked monocytes or macrophages loaded with attenuated *Salmonella* are administered at a single dose, and administration is primarily via intravenous injection.

The cryo-shocked monocytes or macrophages loaded with attenuated *Salmonella* are mainly administered by intravenous injection for treatment, and can also be administered via intraperitoneal injection or intratumoral injection depending on different tumor types.

Compared with injection of cryo-shocked attenuated *Salmonella* alone and a simple mixture of cryo-shocked monocyte line or macrophage line and cryo-shocked attenuated *Salmonella*, the strategy that utilizes the camouflage protection of cryo-shocked monocytes/macrophages to bacteria and targeted delivery to tumor achieves significantly more potent inhibition of tumor growth in mice, along with a marked extension in their survival time. The macrophage line RAW264.7 is taken as an example. Compared with injection of cryo-shocked attenuated *Salmonella* alone, the strategy that utilizes the camouflage protection of cryo-shocked macrophages RAW264.7 to bacteria and targeted delivery to tumor increased the titer of the intratumoral *Salmonella* by 110.9%, decreased the off-target effect of normal organs by 16.3%, and reduced liver lesion regions by 90%.

Attenuated *Salmonella typhimurium* delivered and released from monocyte lines/macrophage lines to tumor tissue are key factors for exerting the anti-tumor efficacy of these drugs.

VNP20009 and the genetically modified strains thereof and the synthetic biologically engineered strains capable of producing therapeutic proteins (including but not limited to aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2; phoP/phoQ), the monocyte lines/macrophage lines with immortalized colonization (including, but not limited to macrophage lines RAW264.7, J774.1, Ana-1, iBMDM, U937, etc. and monocyte lines THP1, iBMMC, J-111, Mono-Mac-1, JOSK-M, etc.) are suitable for such preparation and application solution.

### Example 1

### Preparation of liquid nitrogen cryo-shocked (abbreviated as cryo-shocked hereafter) monocyte line or macrophage line loaded with attenuated Salmonella (cryo-shocked CELL/VNP)

### (1.1) Preparation of liquid nitrogen cryo-shocked (abbreviated as cryo-shocked hereafter) macrophages loaded with attenuated Salmonella (cryo-shocked MACS/VNP)

In order to prepare macrophages loaded with attenuated *Salmonella*, a macrophage line RAW264.7 was co-incubated with attenuated *Salmonella* strains. Specifically, macrophages RAW264.7 in good growth condition were inoculated into a 6-well plate at 5×10⁵ cells/well and cultured with antibiotic-free cell culture medium. Monoclonal strains were picked from the agar plates and activated overnight in LB liquid medium. Bacterial broth grown to log phase is centrifuged at 8000 rpm for 5 min, the supernatant was removed and the sedimented cells were resuspended in sterile saline. After adjusting the OD600 to 1.0, the bacteria were added to 6-well plates plated with the cells described above (MOI 20) and incubated together for different time periods. The attenuated *Salmonella* strains can actively infect cells, while macrophages can also phagocytize the strains **(****FIG. 1a and FIG. 1b****).**

In order to simultaneously observe the morphological change of macrophages during preparation, nuclei were stained with Hoechst (Solarbio, C0030, Beijing, China), and then the morphological change of cells was observed microscopically. The change of breakage percentages of macrophages corresponding to different time points of co-culture of cells and bacteria was recorded. After different time periods, a supernatant was discarded, and the sedimented cells were washed 2-3 times with sterile PBS, and then incubated in the cell culture medium with 75 µg/ml gentamicin for 30 minutes. Gentamicin can kill extracellular strains with no obvious impact on intracellular strains. A supernatant was discarded, and the sedimented cells were washed 2-3 times with sterile PBS. Finally, cells were harvested, and live macrophages loaded with attenuated *Salmonella* strains (live MACS/VNP) were obtained. Due to the self-protection mechanism of the strain VNP20009, the strain loaded in macrophages still retain certain biological activity. To detect the number of the attenuated *Salmonella* effectively loaded in macrophages, the number of live MACS/VNP cells at different time points was detected with the help of a cell counter, and the cells were subsequently lysed with 0.5% Triton X-100 at room temperature. The lysate was diluted and applied to LB agar plates supplemented with kanamycin and incubated overnight at 37°C. The total number of live bacteria loaded in cells was counted. The number of live bacteria loaded in macrophages increased with prolonged co-incubation time **(****FIG. 1c****).** However**,** the i**n**tegrity of macrophages was disrupted as the bacterial loading burden increased **(****FIG. 1c and FIG. 1d****),** which was possibly caused by long-term bacterial loading stimulation. Ultimately, after comprehensively considering the loading capacity of strains and cell integrity, co-culture time of 60 minutes was selected as an optimal time point for preparing live MACS/VNP cells, the load of live bacteria in cells was high at this time **(**every 100 cells were loaded with 257±27 strains), and the cell integrity was high (> 90%) **(****FIG. 1c****).**

Finally, the above prepared live MACS/VNP cells were resuspended with 500-1000 µL of serum-free cell cryopreservation fluid. A cell suspension was snap frozen directly in liquid nitrogen, taken out after 6-18 hours, and then thawed in a water bath at 37-42°C to obtain macrophages cryo-shocked with liquid nitrogen and loaded with the strains VNP20009 (cryo-shocked MACS/VNP cells) **(****FIG. 1a****).**

The present invention also tested VNP20009 and genetically-modified strains thereof and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

### (1.2) Preparation of macrophage line cryo-shocked with liquid nitrogen (abbreviated as cryo-shocked hereafter) and loaded with attenuated Salmonella (cryo-shocked MACS/VNP)

In order to prepare macrophages loaded with attenuated *Salmonella*, another macrophage line J774.1, Ana-1, iBMDM, or U937 replaced the macrophage line RAW264.7, which was co-incubated with attenuated *Salmonella* strains according to operations as described in above (1.1). Specifically, macrophages J774.1, Ana-1, iBMDM, or U937 in good growth condition were inoculated into a 6-well plate at 5×10⁵ cells/well and cultured with antibiotic-free cell culture medium. Monoclonal strains were picked from the agar plates and activated overnight in LB liquid medium. Bacterial broth grown to log phase is centrifuged at 8000 rpm for 5 min, the supernatant was removed and the sedimented cells were resuspended in sterile saline. After adjusting the OD600 to 1.0, the bacteria were added to 6-well plates plated with the cells described above (MOI 20) and incubated together for different time periods. The attenuated *Salmonella* strains can actively infect cells, while macrophages can also phagocytize the strains, and the results were similar to those in **FIG. 1a** and **FIG. 1b**.

In order to simultaneously observe the morphological change of macrophages during preparation, nuclei were stained with Hoechst, and then the morphological change of cells was observed microscopically. The change of breakage percentages of macrophages corresponding to different time points of co-culture of cells and bacteria was recorded. After different time periods, a supernatant was discarded, and the sedimented cells were washed 2-3 times with sterile PBS, and then incubated in the cell culture medium with 75 µg/ml gentamicin for 30 minutes. A supernatant was discarded, and the sedimented cells were washed 2-3 times with sterile PBS. Cells were harvested, and live macrophages J774.1, Ana-1, iBMDM, or U937 loaded with attenuated *Salmonella* strains (live MACS/VNP) were obtained. The number of live MACS/VNP cells at different time points was detected with the help of a cell counter, and the cells were subsequently lysed with 0.5% Triton X-100 at room temperature. The lysate was diluted and applied to LB agar plates supplemented with kanamycin and incubated overnight at 37°C. The total number of live bacteria loaded in cells was counted. The number of live bacteria loaded in the macrophages J774.1, Ana-1, iBMDM, or U937 increased with prolonged co-incubation time. However, the integrity of the macrophages J774.1, Ana-1, iBMDM, or U937 was disrupted as the bacterial loading burden increased. The test results were similar to those in FIG. 1c and FIG. 1d in above (1.1). Co-culture time of 60 minutes was an optimal time point for preparing live MACS/VNP cells, the load of live bacteria in cells was high at this time, and the cell integrity was high (> 90%).

The above prepared live MACS/VNP cells were resuspended with 500-1000 µL of serum-free cell cryopreservation fluid. A cell suspension was snap frozen directly in liquid nitrogen, taken out after 6-18 hours, and then thawed in a water bath at 37-42°C to obtain macrophages cryo-shocked with liquid nitrogen and loaded with the attenuated *Salmonella* strains (cryo-shocked MACS/VNP cells).

The present invention also tested VNP20009 and genetically-modified strains thereof and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

### (1.3) Preparation of monocyte line cryo-shocked with liquid nitrogen (abbreviated as cryo-shocked hereafter) and loaded with attenuated Salmonella (cryo-shocked MC/VNP)

In order to prepare monocytes loaded with attenuated *Salmonella*, a monocyte line THP1, iBMMC, J-111, Mono-Mac-1, or JOSK-M replaced the macrophage line RAW264.7, which was co-incubated with attenuated *Salmonella* strains according to operations as described in above (1.1). Specifically, monocytes THP1, iBMMC, J-111, Mono-Mac-1, or JOSK-M in good growth condition were inoculated into a 6-well plate at 5×10⁵ cells/well and cultured with antibiotic-free cell culture medium. Monoclonal strains were picked from the agar plates and activated overnight in LB liquid medium. Bacterial broth grown to log phase is centrifuged at 8000 rpm for 5 min, the supernatant was removed and the sedimented cells were resuspended in sterile saline. After adjusting the OD600 to 1.0, the bacteria were added to 6-well plates plated with the cells described above (MOI 20) and incubated together for different time periods. The attenuated *Salmonella* strain can actively infect cells, while monocytes can also phagocytize the strains, and the results were similar to those in **(1.1)** and **(1.2)**.

In order to simultaneously observe the morphological change of monocytes during preparation, nuclei were stained with Hoechst, and then the morphological change of cells was observed microscopically. The change of breakage percentages of monocytes corresponding to different time points of co-culturing of cells and bacteria was recorded. After different time periods, a supernatant was discarded, and the sedimented cells were washed 2-3 times with sterile PBS, and then incubated in the cell culture medium with 75 µg/ml gentamicin for 30 minutes. A supernatant was discarded, and the sedimented cells were washed 2-3 times with sterile PBS. Cells were harvested, and live monocytes THP1, iBMMC, J-111, Mono-Mac-1, or JOSK-M loaded with the attenuated *Salmonella* strains (live MC/VNP) were obtained. The number of live MC/VNP cells at different time points was detected with the help of a cell counter, and the cells were subsequently lysed with 0.5% Triton X-100 at room temperature. The lysate was diluted and applied to LB agar plates supplemented with kanamycin and incubated overnight at 37°C. The total number of live bacteria loaded in cells was counted. The number of live bacteria loaded in monocytes THP1, iBMMC, J-111, Mono-Mac-1, or JOSK-M increased with prolonged co-incubation time. However, the integrity of monocytes THP1, iBMMC, J-111, Mono-Mac-1, or JOSK-M was disrupted as the bacterial loading burden increased. The test results were similar to those in above **(1.1)** and **(1.2)**. Co-culture time of 60 minutes was an optimal time point for preparing live MC/VNP cells, the load of live bacteria in cells was high at this time, and the cell integrity was high (> 90%).

The above prepared live MC/VNP cells were resuspended with 500-1000 µL of serum-free cell cryopreservation fluid. A cell suspension was snap frozen directly in liquid nitrogen, taken out after 6-18 hours, and then thawed in a water bath at 37-42°C to obtain macrophages cryo-shocked with liquid nitrogen and loaded with the strains VNP20009 (cryo-shocked MC/VNP cells).

The present invention also tested VNP20009 and genetically-modified strains thereof and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

### Example 2

### Assessment of biological activity of cryo-shocked monocyte line/macrophage line loaded with attenuated Salmonella (CELL/VNP)

### (2.1) Assessment of biological activity of cryo-shocked macrophages loaded with attenuated Salmonella

### (2.1.1) Morphological observation of cryo-shocked MACS/VNP cells and intracellular strains

In order to compare the morphological change of macrophages before and after liquid nitrogen cryo-shock treatment, the above prepared live MACS, live MACS/VNP, and cryo-shocked MACS/VNP cells were photographed with a fluorescence microscope, an SEM, and a TEM, respectively.

For observation of cell morphology with the fluorescence microscope, the live MACS, live MACS/VNP, and cryo-shocked MACS/VNP cells were fixed with 4% paraformaldehyde for 30 minutes and permeabilized with 0.5% Triton X-100 (Sigma-Aldrich, 648462, St. Louis, MO, USA). After washing three times with PBST, Actin-Tracker Green-488 (Beyotime, C2201S) was added for incubation at 37°C for 1 hour, and the cells were stained with DAPI (Beyotime, C1005). Strains VNP-RFP were traced intracellularly with RFP and photographed and observed with the fluorescence microscope (Carl Zeiss, Axioplan 2, Oberkohen, Germany). For observation of cell morphology with SEM, the live MACS, live MACS/VNP, and cryo-shocked MACS/VNP cells were pre-fixed in 2.5% isopropanol at a room temperature for 2 hours and washed with 0.1 M phosphate buffer (pH 7.4). Then, samples were suspended and embedded in 1% agarose, fixed with 1% osmic acid in 0.1 M phosphate buffer (pH 7.4) for 2 hours, and washed several times with 0.1 M phosphate buffer (pH 7.4) at a room temperature. The samples were dehydrated with a series of graded ethanol solutions, dried in a critical point dryer, and finally observed with SEM after gold coating on a sputter coater. For observation of cell morphology with TEM, the live MACS, live MACS/VNP, and cryo-shocked MACS/VNP cells were pre-fixed in 2.5% isopropanol at 4°C for 2-4 hours and washed with 0.1 M phosphate buffer (pH 7.4). Then, samples were suspended and embedded in 1% agarose, fixed with 1% osmic acid in 0.1 M phosphate buffer (pH 7.4) for 2 hours, and washed several times with 0.1 M phosphate buffer (pH 7.4) at a room temperature. After fixation, the samples were dehydrated with a series of graded ethanol, the samples were embedded in Epon812, polymerized in an oven at 60°C for 48 hours, sliced with an ultramicrotome at a thickness of 60-80 nm, and double-stained with 2% uranyl acetate and 2.6% lead citrate for observation with TEM.

The fluorescence microscope **(****FIG. 2a****)** and SEM **(****FIG. 2b****)** images showed that neither loading nor liquid nitrogen cryo-shock treatment of intracellular strains significantly affected the cell integrity, and intact bacteria were observed inside the cells **(****FIG. 2b****).** Therefore, it can be inferred that this strategy of cell loading prior to delivery realizes effective protection characteristics, as bacterial heterogenous substances are no longer directly exposed to environments within organisms.

The above macrophages refer to macrophages of RAW264.7, J774.1, Ana-1, iBMDM, U937, etc. The results of J774.1, Ana-1, iBMDM, and U937 were similar to those of RAW264.7.

The present invention also tested VNP20009 and genetically-modified strains thereof and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

### (2.1.2) Detection of activity and release of intracellular strains in cryo-shocked MACS/VNP cells

Bacteria could achieve cold resistance by regulating the composition of their own cell membranes and the expression of cryo-shock protein, and next, the biological activity of intracellular strains of cryo-shocked MACS/VNP cells was detected. Live MACS/VNP cells and cryo-shocked MACS/VNP cells prepared by the above method were respectively taken at an equal quantity and lysed with 0.5% Triton X-100 at a room temperature. The lysate was diluted and applied to LB agar plates supplemented with kanamycin and incubated overnight at 37°C. The number of monoclonal strains on the plate was counted respectively and compared. The results showed that liquid nitrogen cryo-shock treatment did not affect the activity of the intracellular strains, and the original biological activity of these intracellular strains could be quickly restored after conditions were restored **(****FIG. 2c****).** Subsequently, live MACS/VNP-RFP and cryo-shocked MACS/VNP-RFP cells were prepared according to the above method using the strains VNP20009 that can express RFP (VNP-RFP) available in the laboratory. The prepared two kinds of cells were respectively added to a 96-well plate (1×10⁴ cells/200 µL/well), and the change of fluorescence intensity of RFP (excitation light 550 nm, emission light 585 nm) in each well over incubation time was measured with a microplate reader. Generally, the total fluorescence intensity detected was positively correlated with the number of strains in wells. Significant strain proliferation could be detected in a cryo-shocked MACS/VNP-RFP cell group at the third hour of incubation **(****FIG. 2d****).** Release of strains may be caused by continuous proliferation and movement of intracellular strains. Cold-treated MACS/VNP cells incubated in vitro for different time periods (0/2/4 hours) were fixed with 4% paraformaldehyde, and the change of the state of the intracellular strains was photographed with TEM according to the method as described above. "Release of intracellular bacteria from cells" photographed after incubation for 2 hours and "extracellular proliferation of bacteria" photographed after incubation for 4 hours were observed more intuitively **(****FIG. 2e****).**

These results confirmed that the intracellular attenuated *Salmonella* strains of the cryo-shocked MACS/VNP cells did not lose their biological activity and could be released intracellularly and proliferate quickly under suitable conditions. Compared with the live MACS/VNP group, the strains released earlier in the cryo-shocked MACS/VNP group, indicating that the latter's anti-tumor therapy may achieve quicker strain release and exertion of drug efficacy **(****FIG. 2c****).**

In order to detect the proliferation activity and morphological change of the attenuated *Salmonella* strains released from the cryo-shocked MACS/VNP cells, the intracellular strains were released by lysis of the cells with 0.5% Triton X-100 after obtaining the cryo-shocked MACS/VNP cells. VNP strains in supernatants, namely, released strains were harvested. Two different strains, namely, normal strains and released strains, were subsequently tested for growth curves on an LB medium using Bioscreen C software (OY Growth 175 Curves Ab Ltd., Finland). Briefly, 1 mL of LB medium was infected with 10 µL of attenuated *Salmonella* suspension (OD600 = 1.0), and 300 µL of solution was added to each well of a Bioscreen C multi-well plate. The multi-well plate was incubated at 37°C for 30 hours. OD values were measured at an interval of 30 minutes using a brown filter with a wavelength of 600 nm. Concurrently, fixed samples of the normal strains and released strains were respectively obtained according to the preparation method as described above and photographed with SEM. The results confirmed that the normal strains and released strains had similar proliferation growth characteristics, and could grow to the platform phase at about 24 hours **(****FIG. 3a****).** However, there were no significant differences in the appearance morphology of the two strains **(****FIG. 3b****).**

*Salmonella* can achieve cell infection and movement activity through its flagella, thereby inducing apoptosis or pyroptosis of infected cells. Next, the changes of the capacity of the attenuated *Salmonella* strains released from the cryo-shocked MACS/VNP cells to infect tumor cells and induce apoptosis thereof were respectively detected. For detection of bacterial infection capacity, H22 tumor cells (mouse hepatoma cells) were infected with normal VNP strains and released VNP strains at an MOI of 100 for 1 hour respectively, the cells were washed with PBS after infection, and then 75 µg/mL gentamicin was used for 30 minutes to remove extracellular residual strains. The cells were lysed with 0.5% Triton X-100, and the number of VNP strains internalized was determined by lysing, diluting and coating the cells to an LB plate. For detection of the capacity of bacteria to induce apoptosis, tumor cells (2.0×10⁵) were inoculated to a 12-well plate and subjected to adherent culture for 6-8 hours. Then, the normal attenuated *Salmonella* or released attenuated *Salmonella* strains were harvested and co-cultured with the cells at an MOI of 100 for 4 hours. All cells were taken out from the plate and washed, resuspended in binding buffer, stained with 1 µg of laboratory-made APC-conjugated Annexin V protein, and incubated on ice in the dark for 30 minutes. Finally, 1 µL of propidium iodide (PI, 25 g/mL) was added to all prepared samples, mixed gently, and then detected with a flow cytometer. The results showed that either normal attenuated *Salmonella* or released attenuated *Salmonella* strains could effectively infect tumor cells, with no significant difference in the infection efficiency between the two **(****FIG. 3c****).** In addition, the normal VNP or released attenuated *Salmonella* strains could induce significant apoptosis of tumor cells at levels above 15% compared to merely less than 5% of apoptotic cells in the saline group, with no significant difference between the two **(****FIG. 3d****).**

These results showed that the intracellularly released attenuated *Salmonella* strains were not significantly different from the normal attenuated *Salmonella* strains in terms of growth rate, strain morphology, infection, and induction of tumor cell necrosis. Thus, these released attenuated *Salmonella* strains, when present within tumors, can remodel tumor microenvironments in a classical way, prompting tumor regression. In addition, for better delivery and therapy effects, it is recommended to incubate the cryo-shocked MACS/VNP cells freshly obtained from liquid nitrogen in culture fluid at 37°C for 10-20 minutes to easily restore the biological activity of the intracellular strains before use in subsequent research/therapy.

The above macrophages refer to macrophages of RAW264.7, J774.1, Ana-1, iBMDM, U937, etc. The results of J774.1, Ana-1, iBMDM, and U937 were similar to those of RAW264.7.

The above *Salmonella* refers to VNP20009 and genetically-modified strains thereof, and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

### (2.2) Assessment of biological activity of cryo-shocked monocytes loaded with attenuated Salmonella

### (2.2.1) Morphological observation of cryo-shocked MC/VNP cells and intracellular strains

In order to compare the morphological change of monocytes (MCs) before and after liquid nitrogen cryo-shock treatment, the above prepared live MC, live MC/VNP, and cryo-shocked MC/VNP cells were photographed with a fluorescence microscope, an SEM, and a TEM, respectively.

For observation of cell morphology with the fluorescence microscope, the live MC, live MC/VNP, and cryo-shocked MC/VNP cells were fixed with 4% paraformaldehyde for 30 minutes and permeabilized with 0.5% Triton X-100. After washing three times with PBST, Actin-Tracker Green-488 was added for incubation at 37°C for 1 hour, and the cells were stained with DAPI. Strains VNP-RFP were traced intracellularly with RFP and photographed and observed with the fluorescence microscope. For observation of cell morphology with SEM, the live MC, live MC/VNP, and cryo-shocked MC/VNP cells were pre-fixed in 2.5% isopropanol at a room temperature for 2 hours and washed with 0.1 M phosphate buffer (pH 7.4). Then, samples were suspended and embedded in 1% agarose, fixed with 1% osmic acid in 0.1 M phosphate buffer (pH 7.4) for 2 hours, and washed several times with 0.1 M phosphate buffer (pH 7.4) at a room temperature. The samples were dehydrated with a series of graded ethanol solutions, dried in a critical point dryer, and finally observed with SEM after gold coating on a sputter coater. For observation of cell morphology with TEM, the live MC, live MC/VNP, and cryo-shocked MC/VNP cells were pre-fixed in 2.5% isopropanol at 4°C for 2-4 hours and washed with 0.1 M phosphate buffer (pH 7.4). Then, samples were suspended and embedded in 1% agarose, fixed with 1% osmic acid in 0.1 M phosphate buffer (pH 7.4) for 2 hours, and washed several times with 0.1 M phosphate buffer (pH 7.4) at a room temperature. After fixation, the samples were dehydrated with a series of graded ethanol, the samples were embedded in Epon812, polymerized in an oven at 60°C for 48 hours, sliced with an ultramicrotome at a thickness of 60-80 nm, and double-stained with 2% uranyl acetate and 2.6% lead citrate for observation with TEM.

Observations of the fluorescence microscope and SEM were similar to the results in sections (2.1.1 and 2.1.2) above, which showed that neither loading nor liquid nitrogen cryo-shock treatment of intracellular bacteria significantly affected the cell integrity, and intact bacteria were observed inside the cells.

The above monocytes were THP1, iBMMC, J-111, Mono-Mac-1, or JOSK-M, etc.

The above *Salmonella* refers to VNP20009 and genetically-modified strains thereof, and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria. (2.2.2) Detection of activity and release of intracellular strains of cryo-shocked MC/VNP cells.

Bacteria could achieve cold resistance by regulating the composition of their own cell membranes and the expression of cryo-shock protein, and next, the biological activity of intracellular strains of cryo-shocked MC/VNP cells was detected. Live MC/VNP cells and cryo-shocked MC/VNP cells prepared by the above method were respectively taken at an equal quantity and lysed with 0.5% Triton X-100 at a room temperature. The lysate was diluted and applied to LB agar plates supplemented with kanamycin and incubated overnight at 37°C. The number of monoclonal strains on the plate was counted respectively and compared. The results were similar to those in above (2.1.2), i.e., liquid nitrogen cryo-shock treatment did not affect the activity of the intracellular strains, and the original biological activity of these intracellular strains was quickly restored after conditions were restored. Live MC/VNP-RFP and cryo-shocked MC/VNP-RFP cells were prepared according to the above method using the attenuated *Salmonella* strains that can express RFP (VNP-RFP). The prepared two kinds of cells were respectively added to a 96-well plate (1×10⁴ cells/200 µL/well), and the change of fluorescence intensity of RFP (excitation light 550 nm, emission light 585 nm) in each well over incubation time was measured with a microplate reader. Generally, the total fluorescence intensity detected was positively correlated with the number of strains in wells. Significant strain proliferation could be detected in a cryo-shocked MC/VNP-RFP cell group at the third hour of incubation, with the results similar to those in above (1.2). Release of strains may be caused by continuous proliferation and movement of intracellular strains. Cold-treated MC/VNP cells incubated in vitro for different time periods (0/2/4 hours) were fixed with 4% paraformaldehyde, and the change of the state of the intracellular strains was photographed with TEM according to the method as described above. "Release of intracellular bacteria from cells" after incubation for 2 hours and "extracellular proliferation of bacteria" after incubation for 4 hours were observed intuitively, with the results similar to those in above (2.1.2).

The results confirmed that the intracellular attenuated *Salmonella* strains of the cryo-shocked MC/VNP cells did not lose their biological activity and were released intracellularly and proliferate quickly under suitable conditions. Strains in the cryo-shocked MC/VNP group were released earlier than those in the live MC/VNP group.

In order to detect the proliferation activity and morphological change of the attenuated *Salmonella* strains released from the cryo-shocked MC/VNP cells, the intracellular strains were released by lysis of the cells with 0.5% Triton X-100 after obtaining the cryo-shocked MC/VNP cells. Attenuated *Salmonella* strains in supernatants, namely, released strains were harvested Two different strains, namely, normal strains and released strains, were subsequently tested for growth curves on an LB medium using Bioscreen C software. Briefly, 1 mL of LB medium was infected with 10 µL of VNP20009 (OD600 = 1.0), and 300 µL of solution was added to each well of a Bioscreen C multi-well plate. The multi-well plate was incubated at 37°C for 30 hours. OD values were measured at an interval of 30 minutes using a brown filter with a wavelength of 600 nm. Concurrently, fixed samples of the normal strains and released strains were respectively obtained according to the preparation method as described above and photographed with SEM. The results were similar to those in above (2.1.2), which confirmed that the normal strains and released strains had similar proliferation growth characteristics, and could grow to the platform phase at about 24 hours. However, there were no significant differences in the appearance morphology of the two strains.

*Salmonella* can achieve cell infection and movement activity through its flagella, thereby inducing apoptosis or pyroptosis of infected cells. Next, the changes of the capacity of the attenuated *Salmonella* strains released from the cryo-shocked MS/VNP cells to infect tumor cells and induce apoptosis thereof were respectively detected. For detection of bacterial infection capacity, tumor cells (B16F10, LLC, 4T1, A20, H22) were infected with normal attenuated *Salmonella* strains and released attenuated *Salmonella* strains at an MOI of 100 for 1 hour respectively, the cells were washed with PBS after infection, and then 75 µg/mL gentamicin was used for 30 minutes to remove extracellular residual strains. The cells were lysed with 0.5% Triton X-100, and the number of VNP strains internalized was determined by lysing, diluting and coating the cells to an LB plate. For detection of the capacity of bacteria to induce apoptosis, tumor cells (2.0×10⁵) were inoculated to a 12-well plate and subjected to adherent culture for 6-8 hours. Then, the normal attenuated *Salmonella* or released attenuated *Salmonella* strains were harvested and co-cultured with the cells at an MOI of 100 for 4 hours. All cells were taken out from the plate and washed, resuspended in binding buffer, stained with 1 µg of laboratory-made APC-conjugated Annexin V protein, and incubated on ice in the dark for 30 minutes. Finally, 1 µL of propidium iodide (PI, 25 g/mL) was added to all prepared samples, mixed gently, and then detected with a flow cytometer. The results were similar to those in above (1.2), which showed that either normal attenuated Salmonella or released attenuated Salmonella strains could effectively infect tumor cells, with no significant difference in the infection efficiency between the two. The normal attenuated Salmonella or released attenuated Salmonella strains could induce significant apoptosis of tumor cells at levels above 15% compared to merely less than 5% of apoptotic cells in the saline group, with no significant difference between the two.

These results were similar to those in above (2.1.2), which showed that the intracellularly released attenuated *Salmonella* strains were not significantly different from the normal attenuated *Salmonella* strains in terms of growth rate, strain morphology, infection, and induction of tumor cell necrosis. Thus, these released attenuated *Salmonella* strains, when present within tumors, can remodel tumor microenvironments in a classical way, prompting tumor regression.

The above monocytes were THP1, iBMMC, J-111, Mono-Mac-1, or JOSK-M, etc.

The above *Salmonella* refers to VNP20009 and genetically-modified strains thereof, and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

### Example 3

### Assessment of cell biological activity of cryo-shocked monocyte line/macrophage line loaded with attenuated Salmonella (CELL/VNP)

### (3.1) Detection of cell biological activity of cryo-shocked macrophages loaded with attenuated Salmonella

### (3.1.1) Detection of proliferation capacity of cells in vivo and in vitro

Generally, immortalized cell lines have the capacity to proliferate quickly, so when using such cells as carriers, it is necessary to consider the potential toxic and side effects caused by their strong proliferation capacity to organisms. Next, the proliferation capacity in vivo and in vitro of the macrophage line cryo-shocked with liquid nitrogen was detected. For in vitro proliferation detection, freshly prepared live MACS, cryo-shocked MACS, and cryo-shocked MACS/VNP cells in good condition were selected and inoculated into a 96-well plate at 5×10³ cells per well, 100 µL of completeDMEM was added to each well, and the above cells were cultured in a 5% CO₂ incubator at 37°C and detected with a microplate reader (BioTek) at specific time points. Five replicate wells were set for each time point in each group, original culture liquid in the 96-well plate was replaced with DMEM containing 10% CCK-8 solution (Beyotime Biotechnology Inc., C0039) freshly prepared at specific time, and culture continued for 2 hours. The 96-well plate was taken out and detected with the microplate reader, absorbances of cells in each group were measured at 450 nm, and data results of replicate wells in each group at 0 h, 12 h, 24 h, 36 h, and 48 h were recorded. The test results showed that compared with live MACS cells proliferating rapidly (approximately 75% within 24 hours and approximately 163% within 48 hours), there was no obvious proliferation of cryo-shocked MACS and cryo-shocked MACS/VNP cells within 48 hours. Therefore, it can be basically inferred that cryo-shock treatment deprives macrophage lines of proliferation activity **(****FIG. 4a****).** Subsequently, in vivo pathogenicity experiments were performed using BALB/c mice. Live MACS or cryo-shocked MACS cells were injected subcutaneously into right axillae of the BALB/c mice (1×10⁶ cells per mouse), and photographs were taken every 1-2 days to observe whether there was any mass formed in the axillae. The results showed that in live MACS cells, raised mass was observed as early as Day 5 after injection, which grew larger over time **(****FIG. 4b****).** Correspondingly, mice injected with cryo-shocked MACS cells had no visible mass, and no obvious mass was observed after dissection on Day 14 **(****FIG. 4c****).** This showed that consistent with the in vitro test results, live MACS cells could continue to proliferate in vivo, while cryo-shocked MACS cells lost the activity to reproliferate in vivo.

Obtaining sufficient cells rapidly and conveniently still remains a major challenge in traditional cell therapy. Macrophage lines treated with liquid nitrogen lost their original pathogenicity while ensuring that they were easy and rapid to obtain massively. Therefore, it can be inferred that macrophage lines treated with liquid nitrogen will have broader application value.

The above macrophages refer to macrophages of RAW264.7, J774.1, Ana-1, iBMDM, U937, etc. The results of J774.1, Ana-1, iBMDM, and U937 were similar to those of RAW264.7.

The above *Salmonella* refers to VNP20009 and genetically-modified strains thereof, and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

### (3.1.2) Detection of in vivo tumor-targeted enrichment capacity of cells

Among the inventors' previous published papers and applied patents for anti-tumor research on attenuated *Salmonella*, the most commonly adopted tumor models were B16F10 (melanoma), LLC (lung cancer cells), 4T1 (breast cancer cells), and A20 (lymphoma cells) in sequence; and H22 (hepatoma cells) was the least frequency adopted tumor model. Five tumor models, namely, B16F10, LLC, 4T1, A20, and H22, were adopted in this research.

### Establishment of subcutaneous tumor-bearing mouse models of tumor cells

After the tumor cells grew to an exponential growth phase in DMEM, the cells were harvested and intraperitoneally injected into Balb/c mice (6 weeks old, female, 2×10⁶ cells per mouse), the mice were euthanized after abdominal enlargement of the mice within 3-5 days, and H22 cells were removed from peritoneal fluid of the mice. The obtained tumor cells were washed 2-3 times with PBS, and the cells were resuspended with PBS to regulate the final cell concentration to 1×10⁷ cells/mL. 100 µL of cells were inoculated into an axilla fat pad of each Balb/c mouse, i.e. 1×10⁶ cells/mouse. After inoculation, the mice were housed in a clean animal room, and subsequent experiments were performed when tumor volumes of the mice grew to approximately 80-120 mm³.

### Mode of administration

After randomization of the tumor-bearing mice, different cryo-shocked macrophages were treated with near-infrared (NIR) fluorescent dye DIR (Abbkine, BMD0074, Wuhan, China) for 30 minutes to produce DIR-labeled cells. DIR-labeled cryo-shocked cells were fixed with 4% paraformaldehyde at a room temperature for 1 hour to degenerate and inactivate protein on surfaces of the cells, and fixed cryo-shocked cells were obtained as controls. The above different cells were administered once via tail vein injection with the amount of cryo-shocked VNP strains (1.0×10⁷ cells/mouse) and/or cells (4.0×10⁶ cells/mouse), including cryo-shocked MACS + cryo-shocked VNP (simple mixture of the two), cryo-shocked MACS/VNP, and fixed cryo-shocked MACS/VNP cells. The above strains VNP20009 refer to VNP-LuxCDABE strains established early in the laboratory, which spontaneously emit bioluminescence for in vivo tracing.

The above cells were injected into the tumor-bearing mice via tail veins. Fluorescence signals of LuxCDABE and DIR in mouse tumors treated in different ways were detected with a living imaging system (PerkinElmer, IVIS^{®}Lumina III, Waltham, MA, USA). Significant DIR signals were observed in the cryo-shocked MACS+VNP and cryo-shocked MACS/VNP cell groups 8 hours after injection. Correspondingly, DIR signals in the fixed cryo-shocked MACS/VNP cell group were weak. In addition, the cryo-shocked MACS/VNP cell group had significantly higher LuxCDABE than the other two groups, indicating that it corresponded to a higher strain titer **(****FIG. 5a****).** It can be showed that the tumor-targeted delivery of strains by means of cryo-shocked macrophages has indeed achieved more effective intratumoral enrichment of strains through the tumor-targeted effect of macrophages.

The cryo-shocked cells were subsequently detected for the change of protein content. Cell adhesion molecules such as CD11b and chemokine receptors such as CCR2 play a crucial role in anchoring tumor sites by macrophages. In order to test whether CD11b and CCR2 were still present on the surfaces of the cells, live MAC, live MACS/VNP, and cryo-shocked MACS/VNP cells were harvested and analyzed for expression of CD11b and CCR2 on the surfaces of the cells with a fluorescence microscope and a flow cytometer. For analysis with the fluorescence microscope, the above cells were resuspended in 1% BSA and incubated with protein-specific antibodies, including CD11b antibody (ABclonal, A1581, Wuhan, China) and CCR2 antibody (Proteintech, 16153-1-AP, Wuhan, China). After washing 2-3 times with PBST, a primary antibody was traced with an FITC-labeled fluorescent secondary antibody (Absin, Asp20004, Shanghai, China), and finally the labeling condition was observed with the fluorescence microscope (Carl Zeiss, Axioplan 2, Oberkohen, Germany). For analysis with the flow cytometer, the above cells were resuspended in cell staining buffer, stained with CD11b-APC (BD, 553312) and CCR2-AF647 (Biolegend, cloneSA203G11) for 30 minutes at 4°C in the dark, centrifuged to harvest sedimented cells, and washed 1-2 times with PBS. Fluorescence on the surfaces of the cells was analyzed with the flow cytometer after resuspension with PBS. The results showed that higher CD11b and CCR2 protein content could be detected on the surfaces of the live MACS, live MACS/VNP and cryo-shocked MACS/VNP cells. **(****FIG. 5b and FIG. 5c****)**

Surface integrins and chemokine receptors, including CD11b and CCR2, coupled with the stronger capture effect corresponding to disordered and curved micron-diameter tumor capillaries may be important reasons for efficient aggregation of the cryo-shocked MACS/VNP cells at tumor sites. In conclusion, these results confirmed that the macrophages cryo-shocked with liquid nitrogen described in the present invention could efficiently enrich tumor sites and enable effective delivery of anti-tumor bacteria.

The above macrophages refer to macrophages of RAW264.7, J774.1, Ana-1, iBMDM, U937, etc. The results of J774.1, Ana-1, iBMDM, and U937 were similar to those of RAW264.7.

The results of B16F10, LLC, 4T1, and A20 tumor models were very similar to those of the H22 tumor model.

The above *Salmonella* refers to VNP20009 and genetically-modified strains thereof, and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

### (3.2) Detection of cell biological activity of cryo-shocked monocytes loaded with attenuated Salmonella

### (3.2.1) Detection of proliferation capacity of cells in vivo and in vitro

Generally, immortalized cell lines have the capacity to proliferate quickly, so when using such cells as carriers, it is necessary to consider the potential toxic and side effects caused by their strong proliferation capacity to organisms. Next, the proliferation activity in vivo and in vitro of the monocyte line cryo-shocked with liquid nitrogen was detected. For in vitro proliferation detection, freshly prepared live MC, cryo-shocked MC, and cryo-shocked MC/VNP cells in good condition were selected and inoculated into a 96-well plate at 5×10³ cells per well, 100 µL of complete DMEM was added to each well, and the above cells were cultured in a 5% CO₂ incubator at 37°C and detected with a microplate reader at specific time points. Five replicate wells were set for each time point in each group, original culture liquid in the 96-well plate was replaced with DMEM containing 10% CCK-8 solution freshly prepared at specific time, and culture continued for 2 hours. The 96-well plate was taken out and detected with the microplate reader, absorbances of cells in each group were measured at 450 nm, and data results of replicate wells in each group at 0 h, 12 h, 24 h, 36 h, and 48 h were recorded. The test results were similar to the results in above (3.1.1), which showed that compared with live MC cells proliferating rapidly (approximately 75% within 24 hours and approximately 160% within 48 hours), there was no obvious proliferation of cryo-shocked MC and cryo-shocked MC/VNP cells within 48 hours; and cryo-shock treatment deprives macrophage lines of proliferation activity.

In vivo pathogenicity experiments on cells were performed using BALB/c mice. Live MC or cryo-shocked MC cells were injected subcutaneously into right axillae of the BALB/c mice (1×10⁶ cells per mouse), and photographs were taken every 1-2 days to observe whether there was any mass formed in the axillae. The results were similar to those in above (3.1.1), which showed that in live MC cells, raised mass was observed as early as Day 5 after injection, which grew larger over time. Mice injected with cryo-shocked MACS cells had no visible mass, and no obvious mass was observed after dissection on Day 14. Live MC cells could continuously proliferate in vivo, while cryo-shocked MACS cells lost the activity to reproliferate in vivo.

Monocyte lines treated with liquid nitrogen lost their original pathogenicity while ensuring that they were easy and rapid to obtain massively. Therefore, monocyte lines treated with liquid nitrogen will have broader application value.

The above monocytes were THP1, iBMMC, J-111, Mono-Mac-1, or JOSK-M, etc.

The above *Salmonella* refers to VNP20009 and genetically-modified strains thereof, and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

### (3.2.2) Detection of in vivo tumor-targeted enrichment capacity of cells

Five tumor models, namely, B16F10, LLC, 4T1, A20, and H22, were adopted in this research.

### Establishment of subcutaneous tumor-bearing mouse models of tumor cells

After the tumor cells grew to an exponential growth phase in DMEM, the cells were harvested and intraperitoneally injected into Balb/c mice (6 weeks old, female, 2×10⁶ cells per mouse), the mice were euthanized after abdominal enlargement of the mice within 3-5 days, and H22 cells were removed from peritoneal fluid of the mice. The obtained tumor cells were washed 2-3 times with PBS, and the cells were resuspended with PBS to regulate the final cell concentration to 1×10⁷ cells/mL. 100 µL of cells were inoculated into an axilla fat pad of each Balb/c mouse, i.e. 1×10⁶ cells/mouse. After inoculation, the mice were housed in a clean animal room, and subsequent experiments were performed when tumor volumes of the mice grew to approximately 80-120 mm³.

### Mode of administration

After randomization of the tumor-bearing mice, different cryo-shocked macrophages were treated with near-infrared (NIR) fluorescent dye DIR for 30 minutes to produce DIR-labeled cells. DIR-labeled cryo-shocked cells were fixed with 4% paraformaldehyde at a room temperature for 1 hour to degenerate and inactivate protein on surfaces of the cells, and fixed cryo-shocked cells were obtained as controls. The above different cells were *a*dministered once via tail vein injection with the amount of cryo-shocked attenuated *Salmonella* strains (1.0×10⁷ cells/mouse) and/or cells (4.0×10⁶ cells/mouse), including cryo-shocked MC + cryo-shocked VNP (simple mixture of the two), cryo-shocked MC/VNP, and fixed cryo-shocked MC/VNP cells. The above attenuated *Salmonella* strains used herein are VNP-LuxCDABE strains established in the laboratory, which can spontaneously emit bioluminescence for in vivo tracing.

The above cells were injected into the tumor-bearing mice via tail veins. Fluorescence signals of LuxCDABE and DIR in mouse tumors treated in different ways were detected with a living imaging system. The results were similar to those in above (3.1.2), which showed that significant DIR signals were observed in the cryo-shocked MC+VNP and cryo-shocked MC/VNP cell groups 8 hours after injection. Correspondingly, DIR signals in the fixed cryo-shocked MC/VNP cell group were weak. The cryo-shocked MC/VNP cell group had significantly higher LuxCDABE than the other two groups, indicating that it corresponded to a higher strain titer. The tumor-targeted delivery of strains by means of cryo-shocked macrophages has indeed achieved more effective intratumoral enrichment of strains through the tumor targeting effect of monocytes.

The change of protein content on surfaces of the cryo-shocked cells was subsequently detected according to the method as described in above (3.1.2). Monocytes (such as THP1, iBMMC, J-111, Mono-Mac-1, or JOSK-M), like macrophages, also express cell adhesion molecules such as CD11b and chemokine receptors such as CCR2. In order to test whether CD11b and CCR2 were still present on the surfaces of the cells, live MC, live MC/VNP, and cryo-shocked MC/VNP cells were harvested and analyzed for expression of CD11b and CCR2 on the surfaces of the cells with a fluorescence microscope and a flow cytometer. For analysis with the fluorescence microscope, the above cells were resuspended in 1% BSA and incubated with protein-specific antibodies, including CD11b antibody and CCR2 antibody. After washing 2-3 times with PBST, a primary antibody was traced with an FITC-labeled fluorescent secondary antibody, and finally the labeling condition was observed with the fluorescence microscope. For analysis with the flow cytometer, the above cells were resuspended in cell staining buffer, stained with CD11b-APC and CCR2-AF647 for 30 minutes at 4°C in the dark, centrifuged to harvest sedimented cells, and washed 1-2 times with PBS. Fluorescence on the surfaces of the cells was analyzed with the flow cytometer after resuspension with PBS. The results were similar to those in above (3.1.2), which showed that higher CD11b and CCR2 protein content could be detected on the surfaces of the live MC, live MC/VNP and cryo-shocked MC/VNP cells.

These results confirmed that the liquid nitrogen cryo-shocked monocytes described in the present invention were efficiently enriched in tumor sites and achieved effective delivery of anti-tumor bacteria.

The above monocytes were THP1, iBMMC, J-111, Mono-Mac-1, or JOSK-M, etc.

The results of B16F10, LLC, 4T1, and A20 tumor models were similar to those of the H22 tumor model.

The above *Salmonella* refers to VNP20009 and genetically-modified strains thereof, and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

### Example 4

### Assessment of anti-cancer biological safety of cryo-shocked monocytes/macrophages loaded with attenuated Salmonella (CELL/VNP)

### (4.1) Assessment of anti-cancer biological safety of cryo-shocked macrophages loaded with attenuated Salmonella

Five tumor models, namely, B16F10, LLC, 4T1, A20, and H22, were adopted in this research.

Subcutaneous tumor-bearing mouse models of tumor cells were established with reference to the description in Example 3.

### Mode of administration

After the tumor-bearing mice were randomized, different cells were administered once via tail vein injection with the amount of cryo-shocked VNP strains (1.0×10⁷ cells/mouse) and/or cells (4.0×10⁶ cells/mouse), including cryo-shocked MACS, cryo-shocked VNP strains, cryo-shocked MACS/VNP, and cryo-shocked MACS + cryo-shocked VNP (simple mixture of the two). Tumor-bearing mice only injected with saline were adopted as negative controls.

### Tissue distribution of bacteria on tumor-bearing mice

After 8 hours, 1 day, 3 days, 6 days, and 12 days of administration of the above drugs to the tumor-bearing mice, the mice were randomly sacrificed, and tumors and other organs (including hearts, livers, spleens, lungs, and kidneys) of the tumor-bearing mice were collected in a sterile environment, weighed respectively, and placed in 2 mL of PBS for homogenization with a tissue homogenizer (frequency: 60 Hz; time: 100 s). Different tissues were diluted according to different gradients, coated on an LB plate, and inversely placed in a bacterial incubator at 37°C to be cultured for 12 hours, colonies were counted, and quantity differences of strains VNP20009 in mouse tissues in different groups were compared and analyzed.

In the present invention, titers of bacteria in hearts, livers, spleens, lungs, kidneys, and tumor tissues were tested. High enrichment of bacteria in tumors were observed in the three groups of cryo-shocked MACS, cryo-shocked attenuated *Salmonella* strains, cryo-shocked MACS/VNP, and cryo-shocked MACS + cryo-shocked attenuated *Salmonella*, and there were also different degrees of enrichment in normal organs. Bacteria of three groups reached the highest associated intratumoral strain titer on Day 6. Therefore, Day 6 was described in detail as a representative time point. The bacterial titer in normal organs (including hearts, livers, spleens, lungs, and kidneys) was 4.664 and the bacterial titer in tumors was 7.044 in the cryo-shocked VNP strain group; the bacterial titer in normal organs was 3.903 and the bacterial titer in tumors was 7.813 in the cryo-shocked MACS/VNP group, and the bacterial titer in normal organs was 4.474 and the bacterial titer in tumors was 6.938 in the cryo-shocked MACS + cryo-shocked VNP group **(****FIG. 6a****-****FIG. 6g****).** It could be shown that there was no significant difference in bacterial titers in normal organs and tumor tissues in the cryo-shocked attenuated *Salmonella* strain group and the cryo-shocked MACS + cryo-shocked VNP group. Compared with the above two groups, the bacterial titer in tumor tissues in the cryo-shocked MACS/VNP group was significantly increased while the bacterial titer in normal organs was significantly reduced. This difference between the three groups was also present at other time points. It could be shown that loading prior to delivery of cryo-shocked MACS cells could indeed improve the tumor targeting of attenuated *Salmonella* strains and reduce the off-target for normal organs. This effect could not be achieved only by simply mixing cryo-shocked MACS cells with cryo-shocked VNP strains. The effectiveness of the strategy of the present invention was highlighted.

### Acute toxicity assessment

A total of five groups, including saline, cryo-shocked MACS, cryo-shocked attenuated *Salmonella* VNP strains, cryo-shocked MACS/VNP, and cryo-shocked MACS + cryo-shocked attenuated *Salmonella* VNP, were intravenously administered to H22 tumor-bearing mice respectively, retro-orbital blood of the mice was collected 1 day later (about 200 µL), and whole blood was collected. The whole blood was placed at a room temperature for 30 minutes and then centrifuged at 3000 rpm for 15 minutes. Supernatant serum was collected for IL-6/IL-10 cytokine test and blood biochemistry test including ALT and AST. Mouse cytokine test kit: IL-6 kit (BYabscience, BY-EM220188, Nanjing, China) and IL-10 kit (Liankebio, EK210, Hangzhou, China). At the same time, normal organs (including hearts, livers, spleens, lungs, and kidneys) of the mice were dissected and observed by photographing and by hematoxylineosin (HE) staining **(****FIG. 7a****).**

Observations showed that both the cryo-shocked VNP strains and cryo-shocked MACS + cryo-shocked VNP groups induced significant liver injury, manifested as obvious foci on liver surfaces **(****FIG. 7b****)** and obvious necrotic regions visible by HE staining **(****FIG. 7c****).** Cytokines (IL-6/IL-10) and biochemical indexes (ALT/AST), which were positiv**e**ly correlated with the degree of inflammation in organisms, were significantly increased **(****FIG. 7e and FIG. 7f****).** In contrast, all of the above injury indexes were significantly relieved in the cryo-shocked MACS/VNP group. Specifically, the mean number of acute liver lesions in the saline group was 0, the mean concentrations of IL6 and IL10 in serum were 11.3 pg/mL and 85.8 pg/mL, and the mean concentrations of ALT and AST in serum were 34.5 U/L and 29.3 U/L. The mean number of acute liver lesions in the cryo-shocked VNP strain group was 10, the mean concentrations of IL6 and IL10 in serum were 27.4 pg/mL and 116.0 pg/mL, and the mean concentrations of ALT and AST in serum were 134.9 U/L and 141.3 U/L. The mean number of acute liver lesions in the cryo-shocked MACS + cryo-shocked VNP group was 11, the mean concentrations of IL6 and IL10 in serum were 22.1 pg/mL and 111.3 pg/mL, and the mean concentrations of ALT and AST in serum were 144.3 U/L and 153.1 U/L. The mean number of acute liver lesions in the cryo-shocked MACS/VNP group was 1, the mean concentrations of IL6 and IL10 in serum were 10.9 pg/mL and 94.3 pg/mL, and the mean concentrations of ALT and AST in serum were 55.3 U/L and 59.2 U/L. However, no significant differences were observed in HE of the rest of organs (including hearts, spleens, lungs, and kidneys) in the above groups **(****FIG. 8****).** It could be shown that loading prior to delivery of cryo-shocked MACS cells could indeed reduce toxic and side effects caused by attenuated *Salmonella* strains, improving biological safety. This effect could not be achieved only by simply mixing cryo-shocked MACS cells with cryo-shocked attenuated *Salmonella* strains. The effectiveness of the strategy of the present invention was highlighted.

In order to preliminarily investigate possible reasons for the fact that loading prior to delivery of cryo-shocked MACS cells could indeed reduce toxic and side effects caused by VNP strains and improve intratumoral enrichment of bacteria, the activation condition of immune cells in circulatory system of mice after administration was detected. Considering that neutrophils are powerful main force in removal of foreign bacteria and also main immune cells that cause adverse reactions in organisms, the activation condition of neutrophils is mainly detected. After the mice subjected to different administration therapies were completely anesthetized, whole blood was collected from the mice via eyeball enucleation, and an anticoagulant was added. Peripheral blood lymphocytes were obtained using a peripheral blood lymphocyte separation kit (Solarbio, P8620), and cell aggregates were removed through a 40 µm cell strainer to obtain a single-cell suspension. Cells were stained with the following antimouse antibodies: CD11b-APC (BD, 553312), Ly6G-BV421 (BD, 562737), and CD62L-PE (BD, 553151). Specifically, the collected peripheral blood lymphocytes were resuspended in Hanks buffer containing 1% bovine serum albumin (BSA), and then activated neutrophils in mouse peripheral blood were detected by adding the above CD11b, Ly6G, and CD62L antibodies. After incubation at 4°C for 30 minutes, the cells were washed 2-3 times with buffer, and after unbound antibodies were removed, the cells were loaded onto a flow cytometer for detection. The test results showed that the activation percentage of neutrophils in peripheral blood was higher in the cryo-shocked MACS + cryo-shocked VNP group (approximately 23.5%), while the activation percentage of neutrophils in peripheral blood w**a**s significantly reduced in the cryo-shocked MACS/VNP group (approximately 7.6%) **(****FIG. 9a****).** Activated neutrophils will rapidly remove free bacteria through phagocytosis and secretion of inflammatory factors, and these inflammatory factors will also cause adverse effects on the organisms.

Therefore, by means of the "camouflage protection" effect of the cryo-shocked MACS cells, it could indeed be possible to reduce the potent activation of other immune cells in peripheral blood by reducing massive exposure of strain heterologous substances. This avoids both rapid removal of strains and damage of a large number of rapidly activated immune cells to the organisms **(****FIG. 9b****).** This further verified that the present invention has great potential to facilitate the clinical translation and application of attenuated *Salmonella*.

The above macrophages refer to macrophages of RAW264.7, J774.1, Ana-1, iBMDM, U937, etc. The results of J774.1, Ana-1, iBMDM, and U937 were similar to those of RAW264.7.

The results of B16F10, LLC, 4T1, and A20 tumor models were similar to those of the H22 tumor model.

The above *Salmonella* refers to VNP20009 and genetically-modified strains thereof, and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

### (4.2) Assessment of anti-cancer biological safety of cryo-shocked monocytes loaded with attenuated Salmonella

Five tumor models, namely, B16F10, LLC, 4T1, A20, and H22, were adopted in this research.

Subcutaneous tumor-bearing mouse models of tumor cells were established with reference to the description in Example 3.

### Mode of administration

After the tumor-bearing mice were randomized, different cells were administered once via tail vein injection with the amount of cryo-shocked VNP strains (1.0×10⁷ cells/mouse) and/or cells (4.0×10⁶ cells/mouse), including cryo-shocked MC, cryo-shocked VNP strains, cryo-shocked MC/VNP, and cryo-shocked MC + cryo-shocked VNP (simple mixture of the two). Tumor-bearing mice only injected with saline were adopted as negative controls.

### Tissue distribution of bacteria on tumor-bearing mice

After 8 hours, 1 day, 3 days, 6 days, and 12 days of administration of the above drugs to the tumor-bearing mice, the mice were randomly sacrificed, and tumors and other organs (including hearts, livers, spleens, lungs, and kidneys) of the tumor-bearing mice were collected in a sterile environment, weighed respectively, and placed in 2 mL of PBS for homogenization with a tissue homogenizer (frequency: 60 Hz; time: 100 s). Different tissues were diluted according to different gradients, coated on an LB plate, and inversely placed in a bacterial incubator at 37°C to be cultured for 12 hours, colonies were counted, and quantity differences of attenuated *Salmonella* strains in mouse tissues in different groups were compared and analyzed.

In the present invention, titers of bacteria in hearts, livers, spleens, lungs, kidneys, and tumor tissues were tested, and the results were similar to those in above (4.1). High enrichment of bacteria in tumors were observed in the three groups of cryo-shocked MC, cryo-shocked attenuated *Salmonella* VNP strains, cryo-shocked MC/VNP, and cryo-shocked MC + cryo-shocked VNP, and there were also different degrees of enrichment in normal organs. Bacteria of three groups reached the highest associated intratumoral strain titer on Day 6. On Day 6, in the cryo-shocked attenuated *Salmonella* VNP strain experimental group, the bacterial titer in normal organs (including hearts, livers, spleens, lungs, and kidneys) was approximately 4.5-4.8, and the bacterial titer in tumors was 6.9-7.1; in the cryo-shocked MC/VNP group, the bacterial titer in normal organs was 3.8-4.0, and the bacterial titer in tumors was 7.7-7.9; and in the cryo-shocked MC + cryo-shocked VNP group, the bacterial titer in normal organs was 4.3-4.5, and the bacterial titer in tumors was 6.8-7.0 (the results were similar to those in above 4.1). There was no significant difference in bacterial titers in normal organs and tumor tissues of the cryo-shocked attenuated *Salmonella* VNP strain group and the cryo-shocked MC + cryo-shocked VNP group. The bacterial titer in tumor tissues in the cryo-shocked MC/VNP group was significantly increased while the bacterial titer in normal organs was significantly reduced. This difference between the three groups was also present at other time points. It could be shown that loading prior to delivery of cryo-shocked MC cells could indeed improve the tumor targeting of attenuated *Salmonella* strains and reduce the off-target for normal organs. This effect could not be achieved only by simply mixing cryo-shocked MC cells with cryo-shocked VNP strains.

### Acute toxicity assessment

A total of five groups, including saline, cryo-shocked MC, cryo-shocked VNP strains, cryo-shocked MC/VNP, and cryo-shocked MC + cryo-shocked VNP, were intravenously administered to H22 tumor-bearing mice respectively, retro-orbital blood of the mice was collected 1 day later (about 200 µL), and whole blood was collected. The whole blood was placed at a room temperature for 30 minutes and then centrifuged at 3000 rpm for 15 minutes. Supernatant serum was collected for IL-6/IL-10 cytokine test and blood biochemistry test including ALT and AST. At the same time, normal organs (including hearts, livers, spleens, lungs, and kidneys) of the mice were dissected and observed by photographing and by HE staining.

The results were similar to those in above (4.1): in the cryo-shocked VNP strains and cryo-shocked MC + cryo-shocked VNP groups, obvious foci occurred on liver surfaces, significant liver injury was induced, and obvious necrotic regions were visible by HE staining; and IL-6/IL-10 and ALT/AST were both significantly increased. In the cryo-shocked MC/VNP group, the above injury indexes were significantly relieved. However, no significant differences were observed in HE of the rest of organs (including hearts, spleens, lungs, and kidneys) in the above groups. Therefore, loading prior to delivery of cryo-shocked MC cells could indeed reduce toxic and side effects caused by VNP strains, improving biological safety. This effect could not be achieved only by simply mixing cryo-shocked MC cells with cryo-shocked VNP strains.

In order to investigate possible reasons for the fact that loading prior to delivery of cryo-shocked MC cells indeed reduced toxic and side effects caused by attenuated *Salmonella* strains and improve intratumoral enrichment of bacteria, the activation condition of immune cells in circulatory system of mice after administration was detected. Considering that neutrophils are powerful main force in removal of foreign bacteria and also main immune cells that cause adverse reactions in organisms, the activation condition of neutrophils is mainly detected. After the mice subjected to different administration therapies were completely anesthetized, whole blood was collected from the mice via eyeball enucleation, and an anticoagulant was added. Peripheral blood lymphocytes were obtained using a peripheral blood lymphocyte separation kit, and cell aggregates were removed through a 40 µm cell strainer to obtain a single-cell suspension. The collected peripheral blood lymphocytes were resuspended in Hanks buffer containing 1% bovine serum albumin (BSA), and then activated neutrophils in mouse peripheral blood were detected by adding the above CD11b, Ly6G, and CD62L antibodies. After incubation at 4°C for 30 minutes, the cells were washed 2-3 times with buffer, and after unbound antibodies were removed, the cells were loaded onto a flow cytometer for detection. The test results were similar to those in above (4.1), which showed that the activation percentage of neutrophils in peripheral blood was higher in the cryo-shocked MC + cryo-shocked VNP group (22%-25%), while the activation percentage of neutrophils in peripheral blood was significantly reduced in the cryo-shocked MC/VNP group (7.5%-7.8%). Activated neutrophils will rapidly remove free bacteria through phagocytosis and secretion of inflammatory factors, and these inflammatory factors will also cause adverse effects on the organisms.

Therefore, by means of the "camouflage protection" effect of the cryo-shocked MC cells, it could indeed be possible to reduce the potent activation of other immune cells in peripheral blood by reducing massive exposure of strain heterologous substances. This avoids both rapid removal of strains and damage of a large number of rapidly activated immune cells to the organisms. This further verified that the present invention has great potential to facilitate the clinical translation and application of attenuated *Salmonella*.

The above monocytes were THP1, iBMMC, J-111, Mono-Mac-1, or JOSK-M, etc.

The results of B16F10, LLC, 4T1, and A20 tumor models were similar to those of the H22 tumor model.

The above *Salmonella* refers to VNP20009 and genetically-modified strains thereof, and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

### Example 5

### Assessment of anti-cancer biological safety of cryo-shocked monocytes or macrophages loaded with attenuated Salmonella (CELL/VNP)

### (5.1) Assessment of anti-cancer effect of cryo-shocked macrophages loaded with attenuated Salmonella

Six-week-old female BALB/c mice (Changzhou Animal Center) were selected as experimental animals. Five tumor models, namely, B16F10, LLC, 4T1, A20, and H22, were adopted in this research. Subcutaneous tumor-bearing mouse models of tumor cells were established with reference to the description in Example 3. After tumors grew to approximately 80-120 mm³, mice were randomized to different groups, with 7-8 mice in each group. Mode of administration: different cells were administered once via tail vein injection with the amount of cryo-shocked VNP strains (1.0×10⁷ cells/mouse) and/or cells (4.0×10⁶ cells/mouse), including cryo-shocked MACS, cryo-shocked VNP strains, cryo-shocked MACS/VNP, and cryo-shocked MACS + cryo-shocked VNP (simple mixture of the two). Tumor-bearing mice only injected with saline were adopted as negative controls. The tumor volume was calculated according to the formula V = length × width² × 0.52. Tumor sizes of mice in a specific group were measured at intervals and used to draw a tumor growth curve diagram after calculation. Values were expressed as Mean±SD.

The tumor doubling time was 1.128 days for the saline group, 1.344 days for the cryo-shocked MACS group, 1.403 days for the cryo-shocked VNP20009 strain group, 1.793 days for the cryo-shocked MACS/VNP group, and 1.556 days for the cryo-shocked MACS + cryo-shocked VNP (simple mixture of the two) group **(****FIG. 10a and FIG. 10b**). Compared with the saline group, the tumor doubling time was prolonged by 19.1% in the cryo-shocked MACS group, 24.4% in the cryo-shocked VNP20009 strain group, and 59.0% in the cryo-shocked MACS/VNP group. Compared with the cryo-shocked attenuated *Salmonella* group, the tumor doubling time was prolonged by 10.9% in the cryo-shocked MACS + cryo-shocked VNP (simple mixture of the two) group and 27.8% in the cryo-shocked MACS/VNP group. Compared with the cryo-shocked MACS + cryo-shocked VNP (simple mixture of the two) group, the tumor doubling time was prolonged by 15.2% in the cryo-shocked MACS/VNP group, and the tumor doubling time of cold-treated MACS loaded with VNP strains was 1.15 times that of the theoretical value of the sum of the efficacy of the two, such that the cryo-shocked MACS/VNP group also produced synergistic therapy effects.

After the treated mice were sacrificed on Day 12, tumors were dissected and weighed and photographed. The results showed that compared with the mean tumor weight of the saline group of 1.34 g, the mean tumor weight of the cryo-shocked MACS group was 2.355 g, the mean tumor weight of the cryo-shocked VNP strain group was 0.91 g, the mean tumor weight of the cryo-shocked MACS/VNP group was 0.47 g, and the mean tumor weight of the cryo-shocked MACS + cryo-shocked VNP (simple mixture of the two) group was 0.65 g. The mean tumor weights of the last four groups were reduced to different degrees. The mean tumor weight of the cryo-shocked MACS/VNP group was 72.3% that of the cryo-shocked MACS + cryo-shocked VNP (simple mixture of the two) group (**FIG. 10c and FIG. 10d**). In addition, the survival time of mice corresponding to the cryo-shocked MACS/VNP group was also significantly prolonged compared with the cryo-shocked MACS + cryo-shocked VNP (simple mixture of the two) group (**FIG. 10e**). These further proved that the cryo-shocked MACS/VNP group produced synergistic therapy effects.

The above macrophages refer to macrophages of RAW264.7, J774.1, Ana-1, iBMDM, U937, etc. The results of J774.1, Ana-1, iBMDM, and U937 were similar to those of RAW264.7.

The results of B16F10, LLC, 4T1, and A20 tumor models were similar to those of the H22 tumor model.

The above *Salmonella* refers to VNP20009 and genetically-modified strains thereof, and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

### (5.1) Assessment of anti-cancer effect of cryo-shocked monocytes loaded with attenuated Salmonella

Six-week-old female BALB/c mice (Changzhou Animal Center) were selected as experimental animals. Five tumor models, namely, B16F10, LLC, 4T1, A20, and H22, were adopted in this research. Subcutaneous tumor-bearing mouse models of tumor cells were established with reference to the description in Example 3. After tumors grew to approximately 80-120 mm³, mice were randomized to different groups, with 7-8 mice in each group. Mode of administration: different cells were administered once via tail vein injection with the amount of cryo-shocked VNP strains (1.0×10⁷ cells/mouse) and/or cells (4.0×10⁶ cells/mouse), including cryo-shocked MC, cryo-shocked VNP strains, cryo-shocked MC/VNP, and cryo-shocked MC + cryo-shocked VNP (simple mixture of the two). Tumor-bearing mice only injected with saline were adopted as negative controls. The tumor volume was calculated according to the formula V = length × width² × 0.52. Tumor sizes of mice in a specific group were measured at intervals and used to draw a tumor growth curve diagram after calculation. Values were expressed as Mean ± SD.

The results were similar to those in above (5.1). Compared with the saline group, the tumor doubling time was prolonged by approximately 15% in the cryo-shocked MC group, approximately 20% in the cryo-shocked VNP20009 strain group, and approximately 50% in the cryo-shocked MC/VNP group. Compared with the cryo-shocked attenuated *Salmonella* group, the tumor doubling time was prolonged by approximately 10% in the cryo-shocked MC + cryo-shocked VNP (simple mixture of the two) group and approximately 30% in the cryo-shocked MC/VNP group. Compared with the cryo-shocked MC + cryo-shocked VNP (simple mixture of the two) group, the tumor doubling time was prolonged by 20% in the cryo-shocked MC/VNP group, and the cryo-shocked MACS/VNP group produced synergistic therapy effects.

After the treated mice were sacrificed on Day 12, tumors were dissected and weighed and photographed. The results were similar to those in above (5.1). The cryo-shocked MC/VNP group produced synergistic therapy effects.

The above monocytes (MC) were THP1, iBMMC, J-111, Mono-Mac-1, or JOSK-M, etc.

The results of B16F10, LLC, 4T1, and A20 tumor models were similar to those of the H22 tumor model.

The above *Salmonella* refers to VNP20009 and genetically-modified strains thereof, and synthetic biologically-modified engineered strains capable of producing therapeutic proteins, including aforementioned strains with filed invention applications: ZL201110232776.2, ZL201110220676.8, ZL201410209851.7, ZL201610946268.3, ZL201610945015.4, ZL201610945021.X, ZL202210182222.4, 202010182038.0, 202210070594.8, 202210181929.9, 202210182870.X, 202210182455.4, 202210268084.1, 202210268141.6, 2023102136359; Acta Pharmaceutica Sinica B 2021, 11(10):31653177; Signal Transduction and Targeted Therapy 2023, 8:134; Frontier of Medicine, https://doi.org/10.1007/s11684-022-0925-2;phoP/phoQ. The results showed that the genetically-modified strains of VNP20009 and the synthetic biologically modified engineered strains capable of producing therapeutic proteins showed the same results as those of VNP20009 chassis bacteria.

Basic principles, main features and advantages of the present invention are shown and described above. Those skilled in the art should know that the present invention is not limited by the above experimental examples, the above experimental examples and the specification only describe the principles of the present invention, the present invention may have various changes and improvements on the premise of not departing from the spirit and scope of the present invention, and the scope of protection claimed by the present invention is defined by the appended claims, specification and equivalents thereof.

## Claims

1. A method for preparing cryo-shocked monocytes or macrophages loaded with attenuated *Salmonella*, **characterized in that** it comprises the following steps: co-culturing an immortalized monocyte line or macrophage line with attenuated *Salmonella typhimurium* at an MOI of 1-100 to obtain engineered cells loaded with attenuated *Salmonella*, followed by cryo-shock treatment with liquid nitrogen to produce the cryo-shocked monocytes or macrophages loaded with attenuated *Salmonella*.

2. The method for preparing the cryo-shocked monocytes or macrophages loaded with attenuated *Salmonella* according to claim 1, **characterized in that**: (1) co-incubation of any of monocyte cell lines or macrophage cell lines with attenuated *Salmonella*:
(1) Macrophages or macrophages in good growth condition are plated into a petri dish at 1-100×10⁵ cells per well and cultured using antibiotic-free cell culture medium. Monoclonal strains are picked from the agar plates and activated overnight in LB liquid medium. Bacterial broth grown to log phase is centrifuged at 5000-8000 rpm for 5-10 min, the supernatant is removed and the sedimented cells are resuspended in sterile saline. After adjusting the OD600 to 0.6-1.2, the bacteria are added to petri dishes plated with the cells described above (MOI 1-100) and incubated together for 20-150 min;
(2) To observe morphological changes in cells, cells are observed with the help of a microscope after staining cell nuclei with Hoechst. Changes in the percentage of broken monocytes or macrophages at different time points are recorded. Subsequently, the supernatant is removed and the sedimented cells are washed 2-3 times with sterile PBS and then incubated for 20-60 min using cell culture medium supplemented with 50-125 µg/ml gentamicin. Gentamicin is able to kill extracellular strains. The culture supernatant is removed, and the sedimented cells are resuspended and washed 2-3 times with sterile PBS. Finally, the cells are collected to obtain live monocytes or macrophages loaded with the VNP20009 strain. To detect the number of attenuated *Salmonella* effectively loaded in monocytes or macrophages, the number of live monocytes or macrophages loaded with attenuated *Salmonella* is detected with the help of a cell counter at various time points, and the cells are subsequently lysed with 0.5% Triton X-100 at room temperature. The lysate is diluted and applied to LB agar plates supplemented with kanamycin and incubated overnight at 37°C. The number of viable bacteria loaded in the monocytes or macrophages is counted.
(3) The live cells of the above prepared monocytes or macrophages loaded with attenuated *Salmonella* are resuspended with 500-1000 µL of serum-free cell cryopreservation solution. Subsequently, the cell suspension is snap-frozen directly in liquid nitrogen and left to stand for 6-18 hours before being removed. The cryo-shocked monocytes or macrophages loaded with attenuated *Salmonella* are obtained after thawing in a water bath at 37-42°C.

3. The method for preparing the cryo-shocked monocytes or macrophages loaded with attenuated *Salmonella* according to claim 1 or 2, **characterized in that** in step (1) or step (2), the attenuated *Salmonella typhimurium* is attenuated *Salmonella typhimurium* VNP20009 and genetically modified strains thereof and synthetic biologically engineered strains capable of producing therapeutic proteins, and the immortalized monocyte line/macrophage line comprises any one of THP1, iBMMC, J-111, Mono-Mac-1, JOSK-M, RAW264.7, J774.1, Ana-1, iBMD, or U937; wherein the monocytes comprise any one of monocyte lines THP1, iBMMC, J-111, Mono-Mac-1, or JOSK-M, and the macrophages comprise any one of macrophage lines RAW264.7, J774.1, Ana-1, iBMDM, or U937.

4. The method for preparing the cryo-shocked monocytes or macrophages loaded with attenuated *Salmonella* according to claim 2, **characterized in that** in step (2), after comprehensively considering the bacterial loading capacity and cell integrity, a co-culture time of 60 minutes is selected as an optimal time point for preparing live CELL/VNP cells, due to high live bacterial load, with 257±27 bacteria per 100 cells, and high cell integrity of > 90%.

5. Cryo-shocked monocytes or macrophages loaded with attenuated *Salmonella* prepared by the preparation method according to claim 1.

6. Use of the method for preparing the cryo-shocked monocytes or macrophages loaded with attenuated *Salmonella* according to claim 1 in the preparation of anti-tumor drug formulations.

7. The use according to claim 6, **characterized in that** a therapeutic dose of the cryo-shocked monocytes or macrophages loaded with attenuated *Salmonella* is 0.4-40×10⁶ cells (unit: cells/mouse), and the corresponding actual number of attenuated *Salmonella* is 0.1-10×10⁷ CFU, (unit: CFU/mouse); and the cryo-shocked monocytes or macrophages loaded with attenuated *Salmonella* are administered at a single dose.

8. The use according to claim 6, **characterized in that** the drug formulations comprise at least one of intravenous injections, intratumoral injections, or intraperitoneal injections.

9. Combination of the use according to claim 6 and other conventional anti-tumor drugs or methods.
